(19) Europäisches Patentamt
European Patent Office
Office européen des brevets

(11) Veröffentlichungsnummer: **0 053 767**
**B1**

(12) # EUROPÄISCHE PATENTSCHRIFT

(45) Veröffentlichungstag der Patentschrift: **12.09.84**

(21) Anmeldenummer: **81109949.8**

(22) Anmeldetag: **27.11.81**

(51) Int. Cl.³: **C 07 D 487/04**, C 07 D 471/14, C 07 D 487/14, A 61 K 31/505, A 61 K 31/535 // C07D233/24, (C07D487/04, 239/00, 235/00), (C07D487/04, 239/00, 239/00), (C07D471/14, 239/00, 235/00, 221/00)

(54) **Tricyclische Cytosinderivate zur Verwendung in Arzneimitteln und Verfahren zu ihrer Herstellung.**

(30) Priorität: **09.12.80 DE 3046366**

(43) Veröffentlichungstag der Anmeldung: **16.06.82 Patentblatt 82/24**

(45) Bekanntmachung des Hinweises auf die Patenterteilung: **12.09.84 Patentblatt 84/37**

(84) Benannte Vertragsstaaten: **AT BE CH DE FR GB IT LI NL SE**

(56) Entgegenhaltungen:
**US - A - 3 329 679**

**JOURNAL OF MEDICINAL CHEMISTRY, Band 18, Nr. 5, Mai 1975, Washington, US G.E. HARDTMANN: "Synthesis and Biological Evaluation of some 10-substituted 2,3-dihydroimidazo (2,1-b) quinazolin-5 (10H)-ones, a new class of Bronchodilators", Seiten 447-53**
**DIE PHARMAZIE, Band 34, (H10); Oktober 1979, Berlin, DD G. WAGNER: "Konvalente Bindung verschiedener tetra- und tricyclischer Heterocyclen mit Isothiocyanatstruktur an Humanserum-albumin und Rindergammaglobulin", Seiten 640-645**

(73) Patentinhaber: **BAYER AG, Konzernverwaltung RP Patentabteilung, D-5090 Leverkusen 1 Bayerwerk (DE)**

(72) Erfinder: **Junge, Bodo, Dr., Spiekern 23, D-5600 Wuppertal 23 (DE)**
Erfinder: **Goldmann, Siegfried, Dr., Kuckuckstrasse 41, D-5600 Wuppertal 2 (DE)**
Erfinder: **Thomas, Günter, Dr., Claudiusweg 9, D-5600 Wuppertal 1 (DE)**
Erfinder: **Garthoff, Bernward, Dr., Händelstrasse 22, D-4010 Hilden (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 053 767 B1

ACTORUM AG

**Beschreibung**

Die Erfindung betrifft bekannte tricyclische Cytosinderivate zur Verwendung bei der Bekämpfung von Kreislauferkrankungen, neue Verbindungen aus dieser Stoffklasse zur Verwendung bei der Bekämpfung von Erkrankungen, Verfahren zu ihrer Herstellung, Arzneimittel enthaltend diese Verbindungen sowie Verfahren zur Herstellung dieser Arzneimittel.

Einige der tricyclischen Cytosinderivate sind bereits bekannt geworden [vgl. R.J. Grout et al, J. Chem. Soc. 1960, 3551; S. Leistner et al, Z. Chem. *12*, 289 (1972); G.E. Hardtmann et al, J. Org. Chem. *39*, 3599 (1974); G. Wagner u.a. , Pharmazie 34, 209 (1979), G.E. Hardtmann u.a., J. Med. Chem. 18, 447 (1975)]; für keine dieser Verbindungen ist bisher eine therapeutische Wirksamkeit bekannt geworden.

Für ähnliche stickstoffhaltige tricyclische Verbindungen, die sich chemisch eindeutig von den erfindungsgemässen Verbindungen unterscheiden, sind bereits biologische Wirkungen bekannt. Im US-A-3 329 679 wird z.B. für solche ähnlichen Verbindungen eine Wirkung auf das zentrale Nervensystem beschrieben.

Die Erfindung betrifft tricyclische Cytosinderivate der allgemeinen Formel (I)

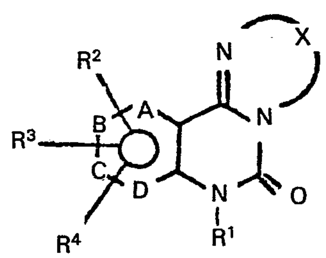

(I)

in welcher

X für ein gesättigtes Brückenglied mit 2 oder 3 Kohlenstoffatomen steht, welches gegebenenfalls durch 1 oder 2 Reste aus der Reihe Alkyl mit 1 bis 4 Kohlenstoffatomen, Phenyl oder Benzyl substituiert ist,

A, B, C und D jeweils für CH stehen, wobei gegebenenfalls 1 oder 2 dieser CH-Gruppen durch N ersetzt sind,

$R^1$ für Wasserstoff, Alkyl, Alkenyl oder Alkinyl mit jeweils bis zu 18 Kohlenstoffatomen steht, wobei die Alkenyl- und Alkinylreste 1, 2 oder 3 Doppel- oder Dreifachbindungen enthalten können, oder für einen mono-, bi- oder tricyclischen aliphatischen Rest mit 3 bis 8 Kohlenstoffatomen, der gesättigt oder einfach oder doppelt ungesättigt sein kann oder für einen gegebenenfalls durch Trifluormethyl, Fluor und Nitro oder Nitro substituierten Phenylrest steht, wobei die genannten Alkyl- und Alkenylreste substituiert sein können durch Substituenten aus der folgenden Gruppe: Hydroxy, Alkoxy mit 1 bis 4 Kohlenstoffatomen, Phenoxy, wobei der Phenylrest durch $NO_2$, Amino, Acetamido, $C_1$-$C_4$- Alkyl, $C_1$-$C_4$-Alkoxy, Halogen $CF_3$, Carboxy oder Carbalkoxy mit 1 bis 4 Kohlenstoffatomen im Alkoxyrest substituiert sein kann; Acyloxy, wobei der Acylrest von aliphatischen Carbonsäuren mit 1 bis 7 C-Atomen oder Phenylcarbonsäuren, die im Phenylrest durch OH, Halogen, $C_1$-$C_4$-Alkyl, $C_1$-$C_4$-Alkoxy, Nitro und/oder Amino substituiert sein können, abgeleitet ist;

Amino, Monoalkylamino und Dialkylamino mit 1 bis 4 Kohlenstoffatomen je Alkylrest, Monoacylamino, wobei der Acylrest von aliphatischen Carbonsäuren mit 1 bis 7 C-Atomen oder Phenylcarbonsäuren, die im Phenylrest durch OH, Halogen, $C_1$-$C_4$-Alkyl, $C_1$-$C_4$-Alkoxy, Nitro und/oder Amino substituiert sein können, abgeleitet ist;

Mercapto, Alkylthio mit 1 bis 4 Kohlenstoffatomen, Halogen;

Alkylcarbonyl mit 1 bis 4 Kohlenstoffatomen im Alkylrest;

Carboxy, Nitro, Cyan, die Aldehydfunktion, die Sulfonsäuregruppe;

Phthalimido, Pyridyl, Thienyl, Furyl, Isoxyzolyl, Thiazolyl;

einen Phenylrest, der einen oder mehrere gleiche oder verschiedene Substituenten aus der Reihe OH, $NH_2$, $C_1$-$C_4$-Alkyl-NH-, $C_1$-$C_4$-Dialkyl-N-, $C_1$-$C_4$-Alkoxy, $NO_2$, CN, COOH, COO-Alkyl ($C_1$-$C_4$), ($C_1$-$C_6$-Alkyl, Chlor oder Brom, $C_1$-$C_4$-Alkylthio, SH, $C_1$-$C_4$-Alkylsulfonyl, $SO_3H$, $SO_2$-$NH_2$, $SO_2$-NH-Alkyl ($C_1$-$C_4$) tragen kann;

$R^2$, $R^3$ und $R^4$ gleich oder verschieden sind und jeweils für Wasserstoff, $C_1$-$C_6$-Alkyl und Alkenyl, Phenyl, $C_1$-$C_6$-Alkoxy, Phenoxy, $C_1$-$C_6$-Alkylthio, Phenylthio, $C_1$-$C_6$-Alkylsulfonyl, Phenylsulfonyl, Acetyl, Benzoyl, Trifluormethyl, Trifluormethoxy, Trifluormethylthio, Hydroxymethyl, Aminomethyl, $C_1$-$C_6$-Alkylcarbonyloxymethyl, $C_1$-$C_4$-Alkylcarbonylaminomethyl, Benzyl, durch OH, Halogen, $C_1$-$C_4$-Alkyl, $C_1$-$C_4$-Alkoxy, Nitro oder Amino substituiertes Benzyl, Phenethyl, Mercapto, Hydroxy, $C_1$-$C_4$-Alkylcarbonyloxy, Benzoyloxy, Amino, $C_1$-$C_4$-Mono- und Dialkylamino, Acylamino, wobei der Acylrest von aliphatischen Carbonsäuren mit 1 bis 12 C-Atomen oder Phenylcarbonsäuren, die im Phenylrest durch OH, Halogen, insbesondere F, Cl, Br, $C_1$-$C_4$-Alkyl, $C_1$-$C_4$-Alkoxy, Nitro oder Amino substituiert sein können, abgeleitet ist;

$C_1$-$C_6$-Alkoxycarbonylamino, einen Harnstoff- oder Thioharnstoffrest, wobei das zweite Stickstoffatom durch $C_1$-$C_{12}$-Alkyl, Phenyl oder durch Phenyl, das durch Halogen, $C_1$-$C_4$-Alkyl, $C_1$-$C_4$-Alkoxy, $CF_3$ oder $NO_2$ substituiert ist, substituiert sein kann;

Sulfonylamino, wobei der Sulfonylrest von aliphatischen Sulfonsäuren mit 1 bis 6 C-Atomen oder Phenylsulfonsäuren, deren Phenylrest durch $NO_2$, Amino, Acetamido, $C_1$-$C_4$-Alkyl, $C_1$-$C_4$-Alkoxy, Halogen, $CF_3$ substituiert sein kann, abgeleitet ist;

Carboxyl, Carbalkoxy von $C_1$-$C_{12}$-Alkoholen abgeleitet;

Carbonamido von Ammoniak, von $C_1$-$C_{12}$ aliphatischen, primären oder sekundären Aminen, von Pyrrolidin, Morpholin oder Piperazin, von Anilin, das durch Halogen, $C_1$-$C_4$-Alkyl, $C_1$-$C_4$-Alkoxy substituiert sein kann;

die Sulfonsäuregruppe, Sulfonamido von $NH_3$, $C_1$-$C_{12}$ primären oder sekundären Aminen, von Pyrrolidin, Piperidin, Piperazin oder Morpholin, von Anilin, das durch Halogen, $C_1$-$C_4$-Alkyl, $C_1$-$C_4$-Alkoxy substituiert sein kann;

F, Cl, Br oder Nitro,

sowie ihre pharmazeutisch verträglichen Salze mit geeigneten organischen oder anorganischen Säuren, wie Chloride, Sulfate, Sulfonate oder Acetate zur Verwendung bei der Bekämpfung von Kreislauferkrankungen.

Die Erfindung betrifft weiterhin neue tricyclische Cytosinderivate der allgemeinen Formel (II)

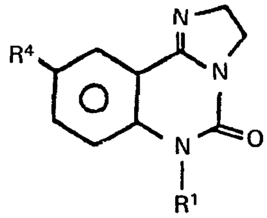

(II)

worin

$R^1$ die oben angegebene Bedeutung hat und

$R^4$ die folgende Bedeutung hat:

Acylamino, wobei der Acylrest von aliphatischen Carbonsäuren mit 1 bis 12 C-Atomen oder Phenylcarbonsäuren, die im Phenylrest durch OH, Halogen, $C_1$-$C_4$-Alkyl, $C_1$-$C_4$-Alkoxy, Nitro oder Amino substituiert sein können, abgeleitet ist;

$C_1$-$C_6$-Alkoxycarbonylamino, einem Harnstoff- oder Thioharnstoffrest, wobei das zweite Stickstoffatom durch $C_1$-$C_{12}$-Alkyl, Phenyl oder durch Phenyl, das durch Halogen, $C_1$-$C_4$-Alkyl, $C_1$-$C_4$-Alkoxy, $CF_3$ oder $NO_2$ substituiert ist, substituiert sein kann;

Sulfonylamino, wobei der Sulfonylrest von aliphatischen Sulfonsäuren mit 1 bis 6 C-Atomen oder Phenylsulfonsäuren, deren Phenylrest durch $NO_2$, Amino, Acetamido, $C_1$-$C_4$-Alkyl, $C_1$-$C_4$-Alkoxy, Halogen, $CF_3$ substituiert sein kann, abgeleitet ist.

Die neuen Verbindungen werden hergestellt, indem man Anthranilsäuren der allgemeinen Formel (III)

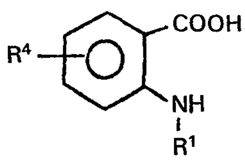

(III)

in welcher

$R^1$ und $R^4$ die oben angegebene Bedeutung haben,

mit einem Chlorkohlensäureester der allgemeinen Formel (IV)

$$ClCOO\text{-}R^5 \qquad (IV)$$

in welcher

$R^5$ für einen gegebenenfalls substituierten Alkyl-, Aryl- oder Aralkylrest steht,

in Gegenwart von inerten aprotischen Lösungsmitteln und unter Zusatz eines säurebindenden Mittels bei Temperaturen zwischen —20 und 150°C umsetzt und das so erhaltene Reaktionsprodukt nach Entfernung des Lösungsmittels mit einem Überschuss eines Diamins der allgemeinen Formel (V)

$$H_2N\text{-}CH_2\text{-}CH_2\text{-}NH_2 \qquad (V)$$

gegebenenfalls in Gegenwart eines inerten aprotischen Lösungsmittels bei Temperaturen zwischen 20 und 150°C umsetzt und anschliessend nach Entfernen von überschüssigem Diamin der Formel (V) und Lösungsmittel den Rückstand in einem inerten hochsiedenden Lösungsmittel bei Temperaturen zwischen 150 und 250°C cyclisiert, wobei in einem anschliessenden Reaktionsschritt gegebenenfalls solche Verbindungen der allgemeinen Formel (II), in welcher $R^1$ Wasserstoff bedeutet, durch übliche Alkylierungsmethoden mit einem Alkylierungsmittel der allgemeinen Formel (VI)

$$R^1\text{-}Z \qquad (VI)$$

in welcher

$R^1$ die oben angegebene Bedeutung besitzt mit Ausnahme von Wasserstoff und

Z für eine für Alkylierungsmittel übliche austretende Gruppe steht, gegebenenfalls unter Phasentransferbedingungen alkyliert werden.

Die Verbindungen der allgemeinen Formel (I) lassen sich in analoger Weise herstellen.

Bei der Herstellung der Verbindungen können anstelle der Anthranilsäuren der allgemeinen Formel (III) und der Kohlensäureester der allgemeinen Formel (IV) auch direkt die Isatosäureanhydride der allgemeinen Formel (VII)

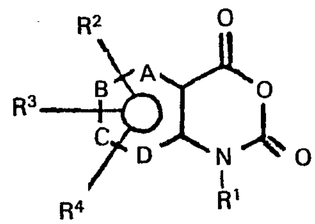

(VII)

in welcher

$R^1$, $R^2$, $R^3$, $R^4$, A, B, C und D die eingangs angegebene Bedeutung haben,

in die Reaktion eingesetzt werden.

Geht man beispielsweise von den Ausgangsstoffen Anthranilsäure, Chlorkohlensäurephenylester und Ethylendiamin aus, so lässt sich das Verfahren durch folgendes Formelschema veranschaulichen:

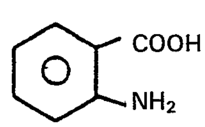

1.

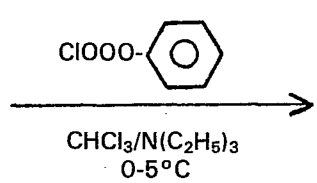


2.

$H_2N$-$CH_2$-$CH_2$-$NH_2$

80°C

3. 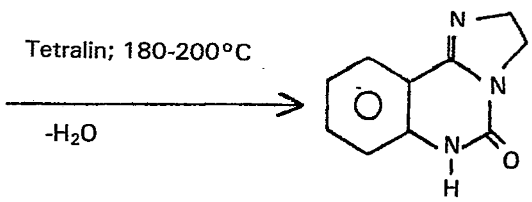


Eine weitere Variante des Verfahrens zur Herstellung solcher Verbindungen, in denen $R^1$ nicht Wasserstoff bedeutet, besteht darin, dass man Verbindungen der allgemeinen Formel (I), in welchen $R^1$ für Wasserstoff steht, nachträglich nach üblichen Methoden alkyliert.

Geht man beispielsweise von 5-Oxo-2,3,5,6-tetrahydroimidazo[1,2-c]chinazolin und Allylbromid als Ausgangsstoffen aus, so lässt sich das Verfahren durch folgendes Formelschema veranschaulichen:

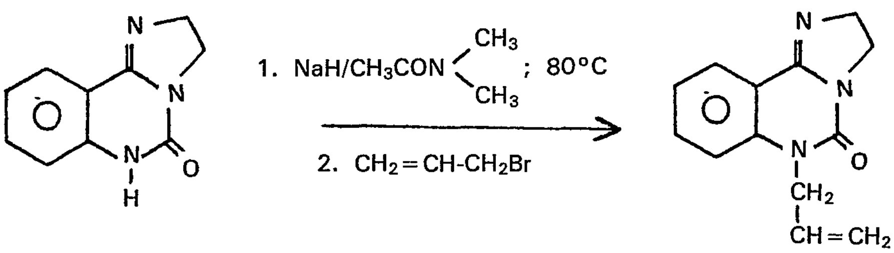


Eine weitere Variante dieses Verfahrens (Alkylierung unter Phasentransferbedingungen) wird durch folgendes Reaktionsschema erläutert:

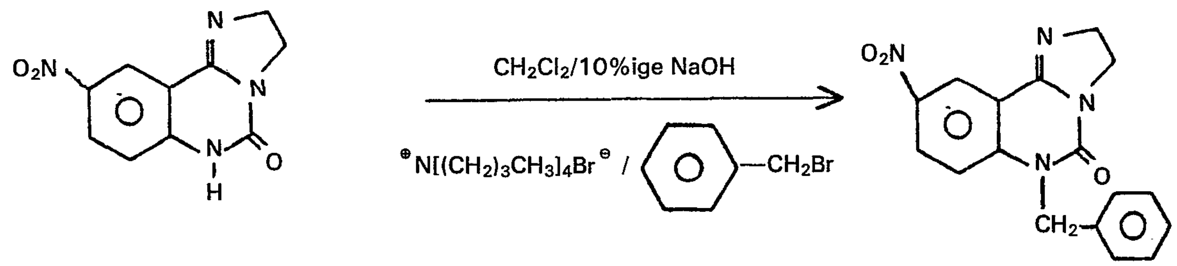


Weiterhin wurde gefunden, dass man ausgehend von Verbindungen der allgemeinen Formel (I), in denen A, B, C und D gleich CH und $R_2$, $R_3$ und $R_4$ = H sind, durch in der Aromatenchemie übliche elektrophile Substitutionsreaktionen zu Verbindungen mit $R_3$ und/oder $R_4 \neq H$ gelangt.

Anhand des folgenden Formelschemas sei dieses Verfahren beispielhaft erläutert:

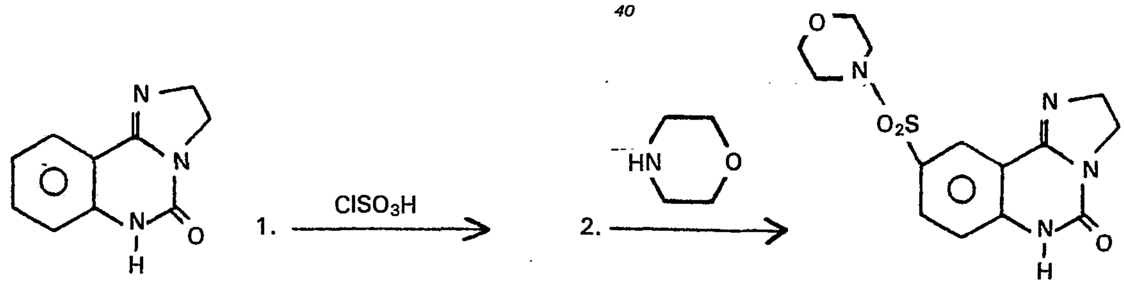


Die so erhaltenen Verbindungen mif funktionellen Resten im Aromaten lassen sich dann, wie das Beispiel zeigt, vielseitig nach üblichen Methoden abwandeln.

Die Umsetzung der Anthranilsäuren (III) mit den Chlorkohlensäureestern (IV) erfolgt in inerten aprotischen Lösungsmitteln unter Zusatz eines säurebindenden Mittels. Als Lösungsmittel seien beispielhaft genannt:

Chloroform, Methylenchlorid, Tetrahydrofuran, Dioxan, Diethylether, Benzol, Toluol.

Als säurebindendes Mittel wird vorzugsweise ein tertiäres Amin, beispielsweise Triethylamin, verwendet. Es können aber auch Alkalicarbonate oder -hydrogencarbonate verwendet werden.

Die Reaktion kann bei Temperaturen zwischen —20 und 150°C, insbesondere bei der Siedetemperatur des Lösungsmittels durchgeführt werden. Bevorzugt wird die Reaktion zwischen 10 und 180°C, insbesondere bei Raumtemperatur, durchgeführt.

Nach Beendigung der Reaktion wird das Lösungsmittel abgezogen oder, wenn das Reaktionsprodukt in dem verwendeten Lösungsmittel schwer löslich ist, wird das Reaktionsprodukt durch Filtration isoliert.

Durch Verrühren mit Wasser werden anschliessend die Salze entfernt und das Zwischenprodukt getrocknet.

Die weitere Umsetzung mit dem Diamin (V) kann mit einem Überschuss des Diamins als Lösungsmittel

erfolgen. Sie kann aber auch mit molaren Mengen des Diamins in einem inerten aprotischen Lösungsmittel durchgeführt werden. Die Reaktion erfolgt zwischen Raumtemperatur und 150°C, bevorzugt zwischen 60 und 100°C.

Anschliessend wird überschüssiges Amin und/oder Lösungsmittel am Rotationsverdampfer entfernt und der Rückstand mit einem inerten hochsiedenden Lösungsmittel versetzt. Vorzugsweise wird hier Tetralin als Lösungsmittel verwendet. Der Kolbeninhalt wird auf Temperaturen zwischen 150 und 250°C, vorzugsweise 180 bis 210°C, erhitzt, wobei Wasser, restliches Diamin und Lösungsmittel, beispielsweise Tetralin, abdestilliert werden.

Zur Alkylierung der Verbindungen der allgemeinen Formel (I) mit $R_1$ = H mit den Alkylierungsmitteln $R_1$-Z wird beispielsweise zunächst mit NaH in einem inerten aprotischen Lösungsmittel wie z.B. Dimethylformamid, Dimethylacetamid, Dimethylsulfoxid oder Hexamethylphosphorsäuretriamid das Anion gebildet und dieses dann mit dem Alkylierungsmittel umgesetzt. Die Bildung des Anions erfolgt bei Temperaturen zwischen 0 und 100°C, vorzugsweise zwischen 40 und 80°C. Die Umsetzung des Anions mit dem Alkylierungsmittel erfolgt zwischen 0 und 100°C, vorzugsweise zwischen Raumtemperatur und 80°C.

Die Alkylierung kann auch in einem Zweiphasensystem unter den Bedingungen einer Phasentransferkatalyse durchgeführt werden. Die eine Phase ist ein inertes, mit Wasser nicht mischbares organisches Lösungsmittel wie beispielsweise Methylenchlorid, Benzol oder Toluol, die andere Phase ist eine wässrige Phase, in der eine starke anorganische Base gelöst ist. Als wässrige Phase kommen beispielsweise 5 bis 40%ige wässrige Natron- oder Kalilauge in Frage, bevorzugt wird eine 10 bis 20%ige Natronlauge verwendet. Die Reaktion wird bei Temperaturen zwischen 20 und 80°C durchgeführt. Als Phasentransferkatalysator kann ein quartäres Ammoniumsalz wie Tetra-n-butylammoniumbromid oder Benzyl-tri-n-butylammoniumbromid verwendet werden.

Die verwendeten Anthranilsäuren, Chlorkohlensäureester, Diamine und reaktiven Alkylierungsmittel sind bekannt oder können nach literaturbekannten Verfahren hergestellt werden (vgl. Rodd's Chemistry of Carbon Compounds, Second Edition, Volume III, Part 6, S. 49 ff.; Houben-Weyl, 4. Auflage, Band 7/4, S. 30 und 31; Houben-Weyl, 4. Auflage, Band 8, S. 75 ff.; Houben-Weyl, 4. Auflage, Band 11/1; Houben-Weyl, 4. Auflage, Band 5/4; Houben-Weyl, 4. Auflage, Band 9, S. 388 und 389).

Überraschenderweise zeigen die erfindungsgemässen Verbindungen der allgemeinen Formel (I) eine Reihe von vorteilhaften pharmakologischen Wirkungen, insbesondere beeinflussen sie den Kreislauf. Im einzelnen bewirken sie an isolierten Herz-Muskel-Präpraten eine starke Steigerung der Kontraktionskraft (positiv inotrope Wirkung) und führen in vivo zu einer beträchtlichen Zunahme der Anspannungsgeschwindigkeit des linken Herzventrikels sowie des Herzschlag- und Herzminutenvolumens, ohne dabei den Blutdruck zu steigern. Sie eignen sich daher insbesondere zur Behandlung von Herzinsuffizienzen. Bei Kenntnis des Standes der Technik konnte nicht erwartet werden, dass diese Stoffklasse eine so vorteilhafte Kreislaufwirkung zeigt.

Zur Erfindung gehören pharmazeutische Zubereitungen, die neben nicht-toxischen, inerten pharmazeutisch geeigneten Trägerstoffen eine oder mehrere erfindungsgemässe Verbindungen enthalten oder die aus einer oder mehreren dieser Verbindungen bestehen, sowie Verfahren zur Herstellung dieser Zubereitungen.

Zur Erfindung gehören auch pharmazeutische Zubereitungen in Dosierungseinheiten. Dies bedeutet, dass die Zubereitungen in Form einzelner Teile, z.B. Tabletten, Dragees, Kapseln, Pillen, Suppositorien und Ampullen, vorliegen, deren Wirkstoffgehalt einem Bruchteil oder einem Vielfachen einer Einzeldosis entsprechen. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben Tagesdosis oder einem Drittel oder einem Viertel einer Tagesdosis entspricht.

Unter nicht-toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten. Dragees, Kapseln, Pillen, Granulate, Suppositorien, Lösungen, Suspensionen und Emulsionen, genannt.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzucker, Glukose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumbicarbonat, (e) Lösungsverzögerer, z.B. Paraffin und (f) Resorptionsbeschleuniger, z.B. quarternäre Ammoniumverbindungen, (g) Netzmittel, z.B. Cetylalkohol, Glycerinmonostearat, (h) Adsorptionsmittel, z.B. Kaolin und Bentonit und (i) Gleitmittel, z.B. Talkum-, Calcium- und Magnesiumstearat und feste Polyethylenglykole oder Gemische der unter (a) bis (i) aufgeführten Stoffe.

Die Tabletten, Dragees, Kapseln, Pillen und Granulate können mit den üblichen, gegebenenfalls Opakisierungsmittel enthaltenden Überzügen und Hüllen versehen sein und auch so zusammengesetzt sein, dass sie den oder die Wirkstoffe nur oder bevorzugt in einem bestimmten Teil des Intestinaltraktes, gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyethylenglykole, Fette, z.B. Kakaofett und höhere Ester (z.B. $C_{14}$-Alkohol mit $C_{16}$-Fettsäure) oder Gemische dieser Stoffe.

Lösungen und Emulsionen können neben dem oder

den Wirkstoffen die üblichen Trägerstoffe, wie Lösungsmittel, Lösungsvermittler und Emulgatoren, z.B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnussöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe, wie flüssige Verdünnungsmittel, z.B. Wasser, Ethylalkohol, Propylenglykol, Suspendiermittel, z.B. ethoxylierte Isostearylalkohole, Polyoxyethylensorbit- und sorbitanester, mikrokristalline Cellulose, Aluminiummethanhydroxid, Bentonit, Agar-Agar und Traganth oder Gemische dieser Stoffe enthalten.

Die genannten Formulierungsformen können auch Färbemittel, Konservierungsstoffe sowie geruchs- und geschmacksverbessernde Zusätze, z.B. Pfefferminzöl und Eukalyptusöl und Süssmittel, z.B. Saccharin, enthalten.

Die therapeutisch wirksamen Verbindungen sollen in den oben aufgeführten pharmazeutischen Zubereitungen in einer Konzentration von etwa 0,1 bis 99,5, vorzugsweise von etwa 0,5 bis 95 Gew.-% der Gesamtmischung, vorhanden sein.

Die oben aufgeführten pharmazeutischen Zubereitungen können ausser den erfindungsgemässen Verbindungen auch andere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Zur Erfindung gehört auch die Verwendung der erfindungsgemässen Verbindungen sowie die Verwendung von pharmazeutischen Zubereitungen, die eine oder mehrere dieser Verbindungen enthalten, in der Human- und Veterinärmedizin zur Verhütung, Besserung und/oder Heilung der oben angeführten Erkrankungen.

Der Wirkstoffe oder die pharmazeutischen Zubereitungen können oral, parenteral, intraperitoneal und/oder rektal, vorzugsweise oral, in einer magensaftresistenten Zubereitung appliziert werden.

Im allgemeinen hat es sich sowohl in der Humanals auch in der Veterinärmedizin als vorteilhaft erwiesen, den oder die Wirkstoffe in Mengen von etwa 0,1 bis etwa 100, vorzugsweise 1,0 bis 50 mg/kg Körpergewicht je 24 Stunden, verteilt auf 1 bis 6 Verabreichungen und zwar vor oder/und während oder/ und nach der Mahlzeit oral zu applizieren. Eine Einzelgabe enthält den oder die Wirkstoffe, vorzugsweise in Mengen von etwa 1,0 bis etwa 20 mg/kg Körpergewicht. Es kann jedoch erforderlich sein, von den genannten Dosierungen abzuweichen und zwar in Abhängigkeit von der Art und dem Körpergewicht des zu behandelden Objekts, der Art und der Schwere der Erkrankung, der Art der Zubereitung und der Applikation des Arzneimittels sowie dem Zeitraum bzw. Intervall, innerhalb welchem die Verabreichung erfolgt. So kann es in einige Fällen ausreichend sein, mit weniger als der o.g. Menge Wirkstoff auszukommen, während in anderen Fällen die oben angeführte Wirkstoffmenge überschritten werden muss. Die Festlegung der jeweils erforderlichen optimalen Dosierung und Applikationsart der Wirkstoffe kann durch jeden Fachmann aufgrund seines Fachwissens leicht erfolgen.

Die positiv inotrope Wirkung der Verbindungen der allgemeinen Formel (I) wurde an Meerschweinchenvorhöfen und isoliert perfundierten Meerschweinchenherzen gefunden. Dazu wurden nach Töten der Tiere durch Nackenschlag und Exzission der Herzen die linken Vorhöfe in ein bei 30 °C thermostatisiertes Bad mit carbogenbegaster Krebs-Henseleit-Lösung eingehängt und die isometrische Spannungsentwicklung der mit 1 Hertz elektrisch stimulierten Vorhöfe vor und 14 Minuten nach Zugabe der zu untersuchenden Substanzen über einen Statham-Druckaufnehmer registriert. Die Untersuchungen an isoliert perfundierten Meerschweinchenherzen erfolgten modifiziert nach Opie [L. Opie, J. Physiol. *180* (1965). 529-541]. Die Herzen wurden retrograd durch die Aorta und die Koronararterien mit 10 ml carbogengesättigter Krebs-Henseleit-Lösung pro Minute perfundiert, und, nach Einführen eines flüssigkeitsgefüllten Latexballons, der mit einem Statham-Druckaufnehmer verbunden war, in den linken Ventrikel, die isovolumetrische Spannungsentwicklung gemessen. Es wurde gefunden, dass die erfindungsgemässen Verbindungen der allgemeinen Formel (I) im Bereich von $10^{-7}$ bis $10^{-4}$ g/ml an den oben beschriebenen isolierten Organen in Gegenwart und in Abwesenheit des Betarezeptorenblockers Propranolol positiv inotrop wirken.

An der mit Pentobarbital narkotisierten Katze steigern diese Verbindungen die Anspannungsgeschwindigkeit des linken Herzventrikels, insbesondere auch nach intraduodenaler Verabreichung im Bereich von 0,1 bis 10 mg/kg. Dabei wird der systemische Blutdruck nicht erhöht, sondern sogar leicht gesenkt. Darüber hinaus führen die erfindungsgemässen Verbindungen der allgemeinen Formel (I) auch zu einer Thrombozytenaggregationshemmung, was bei Herzinsuffizienz aufgrund ischämischer Herzbezirke vorteilhaft sein kann.

Diese vorteilhaften Wirkungen stellen somit eine Bereicherung der Pharmazie dar.

*Beispiel 1*

*5-Oxo-2,3,5,6-tetrahydroimidazo[1,2-c]chinazolin*

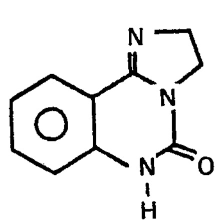

46 g (0,33 Mol) Anthranilsäure und 45 ml Triethylamin werden in 300 ml $CHCl_3$ gelöst und bei einer Temperatur zwischen 0 und 5 °C tropfenweise mit einer Lösung von 57 g (0,33 Mol + 10%) Chlor-

ameisensäurephenylester in 100 ml $CHCl_3$ versetzt. Das Reaktionsgemisch wird über Nacht bei Raumtemperatur gerührt. Dann zieht man das Lösungsmittel am Rotationsverdampfer ab, ohne von ausgefallenem Produkt abzutrennen. Der Rückstand wird mit Wasser ausgerührt, dann abgesaugt. Nach dem Trocknen wird das Rohprodukt (76 g) für die weitere Umsetzung verwendet. 76 g des Rohprodukts werden portionsweise zu 150 ml Ethylendiamin gegeben, dann wird noch 20 Minuten bei 80°C nachgerührt. Das überschüssige Ethylendiamin wird am Rotationsverdampfer abgezogen und der Rückstand mit 150 ml Tetralin versetzt. Man erhitzt das Gemisch in einem Ölbad (Temperatur: 200 bis 240°C) und destilliert sich bildendes Wasser, überschüssiges Ethylendiamin und Tetralin ab, bis die Innentemperatur auf ca. 190°C gestiegen ist (30 bis 60 Minuten). In die noch warme Lösung tropft man 100 ml Ethanol und lässt auskristallisieren. Der Niederschlag wird abgesaugt, mit Alkohol und Ether nachgewaschen und mit 150 ml DMF 1 Stunde lang ausgerührt. Man saugt erneut ab, wäscht mit Alkohol nach und trocknet.

Ausbeute: 25 g = 40% (bezogen auf eingesetzte Anthranilsäure);

Fp.: 295°C.

Zur weiteren Reinigung kann aus DMF umkristallisiert werden. Fp.: 298 bis 300°C.

Analog Beispiel 1 werden hergestellt:

*Beispiel 2*

*9-Chlor-5-oxo-2,3,5,6-tetrahydroimidazo[1,2-c]-chinazolin*

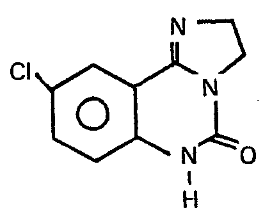

Fp.: >300°C;

*Beispiel 3*

*9-Fluor-5-oxo-2,3,5,6-tetrahydroimidazo[1,2-c]-chinazolin*

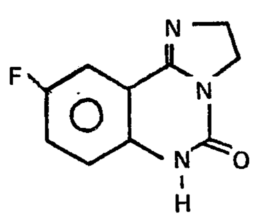

Fp.: >310°C;
Ausbeute: 41%

*Beispiel 4*

*10-Chlor-5-oxo-2,3,5,6-tetrahydroimidazo[1,2-c]-chinazolin*

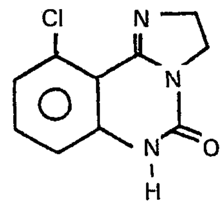

Fp.: 330°C;

*Beispiel 5*

*7,9-Dichlor-5-oxo-2,3,5,6-tetrahydroimidazo[1,2--c]chinazolin*

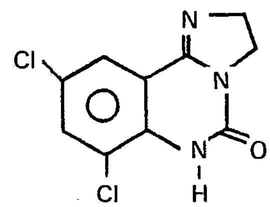

Fp.: 298-299°C;

*Beispiel 6*

*9-Methyl-5-oxo-2,3,5,6-tetrahydroimidazo[1,2-c]-chinazolin*

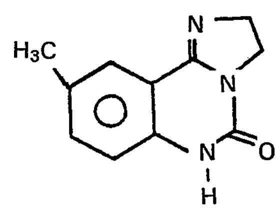

Fp.: 310-313°C

*Beispiel 7*

*8,10-Dimethyl-5-oxo-2,3,5,6-tetrahydroimidazo-[1,2-c]chinazolin*

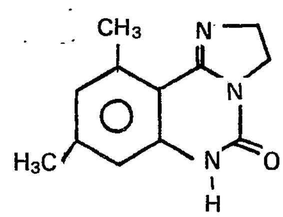

Fp.: 270°C

*Beispiel 8*

*9-Trifluormethyl-5-oxo-2,3,5,6-tetrahydroimidazo-[1,2-c]chinazolin*

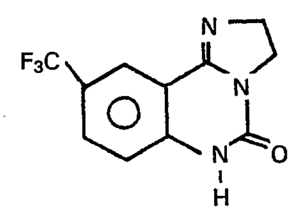

Fp.: 295-298°C;
Ausbeute: 23%

*Beispiel 9*

*8-Trifluormethyl-5-oxo-2,3,5,6-tetrahydroimidazo-[1,2-c]chinazolin*

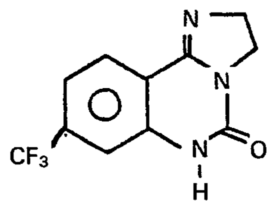

Fp.: > 310°C
Ausbeute: 10%

*Beispiel 10*

*9-Trifluormethoxy-5-oxo-2,3,5,6-tetrahydroimidazo[1,2-c]chinazolin*

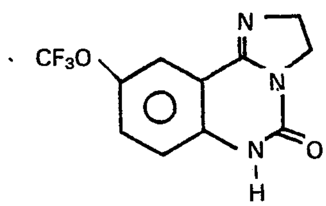

Fp.: 272-274°C
Ausbeute: 42%

*Beispiel 11*

*8,9-Dimethoxy-5-oxo-2,3,5,6-tetrahydroimidazo-[1,2-c]chinazolin*

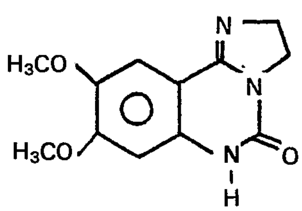

Fp.: 283°C;
Ausbeute: 36%

*Beispiel 12*

*7,8,9-Trimethoxy-5-oxo-2,3,5,6-tetrahydroimidazo[1,2-c]chinazolin*

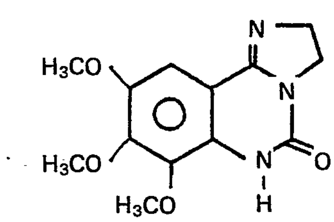

Fp.: 237°C;

*Beispiel 13*

*6-Methoxy-9-chlor-10-methyl-5-oxo-2,3,5,6-tetrahydroimidazo[1,2-c]chinazolin*

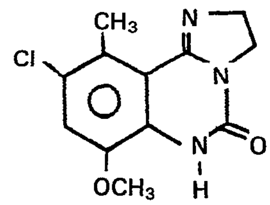

Fp.: 280-286°C;

*Beispiel 14*

*5-Oxo-2,3,5,6-tetrahydroimidazo[1,2-c]pyrido-[3,2-e]-pyrimidin*

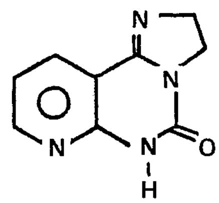

Fp.: 260°C;
Ausbeute: 10%

*Beispiel 15*

*5-Oxo-2,3,5,6-tetrahydroimidazo[1,2-c]pyrido-[2,3-e]pyrimidin*

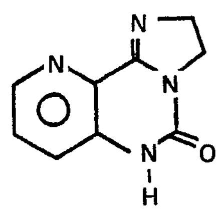

*Fp.:* > 280°C;
Ausbeute: 18%

*Beispiel 16*

*7-Methoxy-8-methyl-5-oxo-2,3,5,6-tetrahydroimidazo[1,2-c]chinazolin*

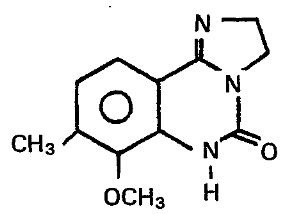

Fp.: 205-210°C;

*Beispiel 17*

*7,9-Dibrom-5-oxo-2,3,5,6-tetrahydroimidazo-[1,2-c]chinazolin*

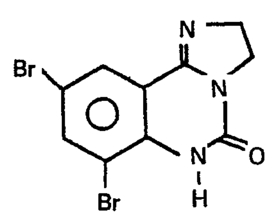

Fp.: 315°C;
Ausbeute: 43%

*Beispiel 18*
*7-Methyl-5-oxo-2,3,5,6-tetrahydroimidazo[1,2-c]-chinazolin*

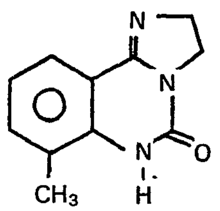

Fp.: 250-255°C;
Ausbeute: 26%

*Beispiel 19*
*5-Oxo-6-allyl-2,3,5,6-tetrahydroimidazo[1,2-c]-chinazolin*

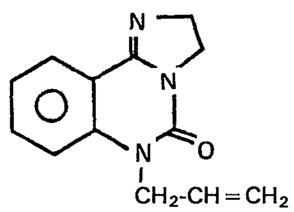

1,75 g (0,058 Mol) einer 80%igen Suspension von NaH in Öl (Schuchardt) werden nach zweimaligem Ausrühren mit je 20 ml n-Hexan und Abdekantieren des Lösungsmittels mit 6,9 g (0,037 Mol) 5-Oxo-2,3,5,6-tetrahydroimidazo[1,2-c]chinazolin in 60 ml absolutem Dimethylacetamid versetzt und 1 Stunde auf 80°C erhitzt. Dabei entsteht allmählich eine klare Lösung. Man kühlt ab und tropft 4,6 ml (0,053 Mol) Allylbromid zu. Dann erhitzt man 4 Stunden auf 100°C. Man engt am Rotationsverdampfer auf ein Drittel des Volumens ein und giesst auf Eiswasser. Das ausgefallene Produkt wird abgesaugt und aus Methanol/Wasser umkristallisiert.
Ausbeute: 4 g (48% der Theorie);
Fp.: 128-131°C.
Analog Beispiel 19 wurden hergestellt:

*Beispiel 20*
*5-Oxo-6-methyl-2,3,5,6-tetrahydroimidazo[1,2-c]-chinazolin*

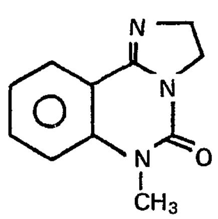

Fp.: 159-163°C;
Ausbeute: 44%

*Beispiel 21*
*5-Oxo-6-n-butyl-2,3,5,6-tetrahydroimidazo [1,2-c]chinazolin*

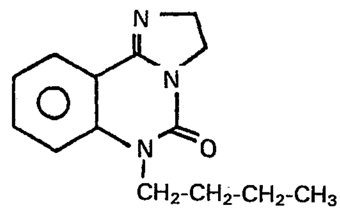

Fp.: 113-115°C;
Ausbeute: 31%

*Beispiel 22*
*5-Oxo-6-diethylaminoethyl-2,3,5,6-tetrahydroimidazo[1,2-c]chinazolin*

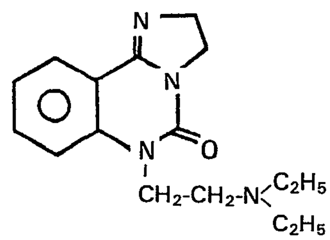

Fp.: 54-57°C;
Ausbeute: 35%

*Beispiel 23*
*5-Oxo-6-benzyl-2,3,5,6-tetrahydroimidazo[1,2-c]-chinazolin*

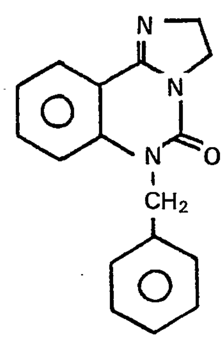

Fp.: 142-144°C;
Ausbeute: 36%

*Beispiel 24*
*5-Oxo-6-[3,4-dichlor-α-methyl-benzyl]-2,3,5,6-tetrahydroimidazo[1,2-c]chinazolin*

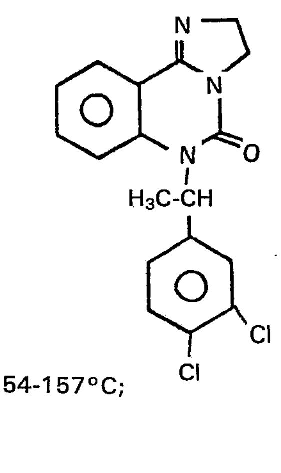

Fp.: 154-157°C;

*Beispiel 25*

*9-Nitro-5-oxo-6-methyl-2,3,5,6-tetrahydroimidazo[1,2-c]chinazolin*

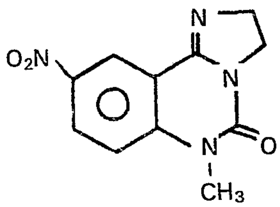

Fp.: 259-260°C;
Ausbeute: 58%
(Als Ausgangsmaterial wurde 9-Nitro-5-oxo-2,3,5,6-tetrahydroimidazo[1,2-c]chinazolin verwendet).

*Beispiel 26*

*5-Oxo-6-methyl-2,3,5,6-tetrahydroimidazo[1,2-c]-pyrido[2,3-e]pyrimidin*

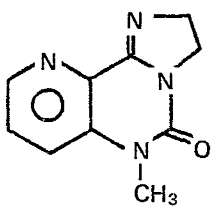

Fp.: 247-250°C;
Ausbeute: 37%

*Beispiel 27*

*5-Oxo-6-allyl-2,3,5,6-tetrahydroimidazo[1,2-c]-pyrido[2,3-e]pyrimidin*

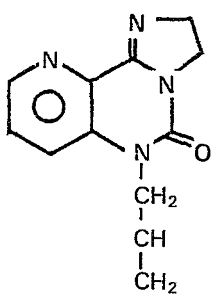

Fp.: 236°C;
Ausbeute: 20%

*Beispiel 28*

*5-Oxo-6-benzyl-2,3,5,6-tetrahydroimidazo[1,2-c]-pyrido[2,3-e]pyrimidin*

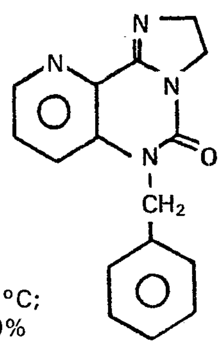

Fp.: 205-207°C;
Ausbeute: 19%

*Beispiel 29*

*9-Nitro-5-oxo-6-benzyl-2,3,5,6-tetrahydroimidazo-[1,2-c]chinazolin*

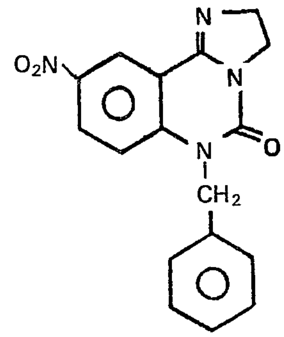

11,5 g (0,05 Mol) 9-Nitro-5-oxo-2,3,5,6-tetrahydroimidazo[1,2-c]chinazolin und 6,4 g (0,02 Mol) Tetra-n-butylammoniumbromid werden in 250 ml 10%iger Natronlauge suspendiert. Dazu gibt man 17,815 g (0,15 Mol) Benzylbromid in 150 ml Methylenchlorid und rührt 2 Stunden bei Raumtemperatur.

Die organische Phase wird abgetrennt und die wässrige Phase 2 mal mit $CH_2Cl_2$ extrahiert. Die vereinigten organischen Phasen werden 2 mal mit wenig Wasser gewaschen und getrocknet. Nach Entfernen des Lösungsmittels wird der Rückstand mit Ethanol kristallisiert und abgesaugt. Die Kristalle werden aus Methanol umkristallisiert.

Ausbeute: 8 g (50% der Theorie);
Fp.: 175-177°C.

Analog Beispiel 29 wurde hergestellt:

*Beispiel 30*

*9-Nitro-5-oxo-6-methyl-2,3,5,6-tetrahydroimid azo[1,2-c]chinazolin*

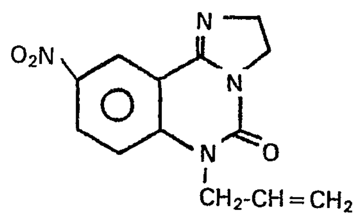

Fp.: 207-209°C;
Ausbeute: 81%

*Beispiel 31*

*9-Nitro-5-oxo-2,3,5,6-tetrahydroimidazo[1,2-c] chinazolin*

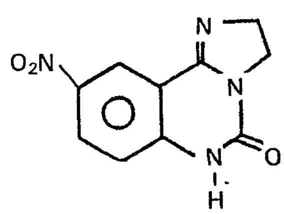

Zu 5 ml 98%iger $HNO_3$ gibt man unter Kühlung 7 ml konzentrierter $H_2SO_4$. Zu dieser Mischung gibt man portionsweise bei 0 bis 5°C 9,3 g (0,05 Mol) 5-Oxo-2,3,5,6-tetrahydroimidazo[1,2-c]chinazolin

und lässt 2 Stunden bei Raumtemperatur rühren. Anschliessend wird das Reaktionsgemisch auf Eiswasser gegeben und der ausgefallene Niederschlag wird abgesaugt. Er wird in Wasser mit $NaHCO_3$ neutralisiert, wobei kurz zum Sieden erhitzt wird. Nach dem Abkühlen wird der Niederschlag erneut abgesaugt, mit Ethanol und Ether gewaschen und getrocknet.

Ausbeute: 8,3 g (72% der Theorie);
Fp.: > 300°C

*Beispiel 32*

*7,9-Dinitro-5-oxo-2,3,5,6-tetrahydroimidazo-[1,2-c]chinazolin*

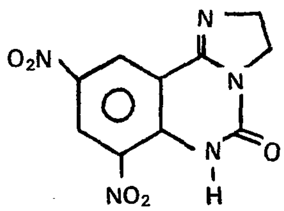

Wird die Reaktionszeit unter den oben bei Beispiel 31 gegebenen Bedingungen auf 24 Stunden ausgedehnt, so erhält man hauptsächlich die Dinitroverbindung.

Fp.: 225-226°C;

*Beispiel 33*

*9-Amino-5-oxo-2,3,5,6-tetrahydroimidazo[1,2-c]-chinazolin*

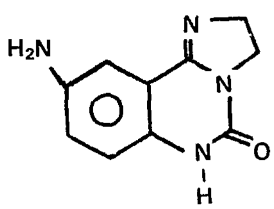

In 4,8 g (0,12 Mol) NaOH in 80 ml $H_2O$ werden 4,6 g 9-Nitro-5-oxo-2,3,5,6-tetrahydroimidazo-[1,2-c]chinazolin gelöst. Man gibt 10,5 g Natriumdithionit zu, wobei die Temperatur auf 60°C steigt. Anschliessend erhitzt man noch kurz zum Sieden. Nach dem Abkühlen werden die ausgefallenen Kristalle abgesaugt, mit $H_2O$ gewaschen und schliesslich in 1 n NaOH ausgerührt. Dann wird erneut abgesaugt, mit Wasser, Ethanol und Ether gewaschen und getrocknet.

Ausbeute: 3,3 g (82% der Theorie);
Fp.: > 300°C.

Analog Beispiel 33 wurden hergestellt:

*Beispiel 34*

*9-Amino-5-oxo-6-allyl-2,3,5,6-tetrahydroimidazo-[1,2-c]chinazolin*

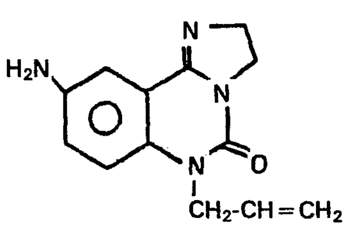

Fp.: 183-184°C;
Ausbeute: 31%

*Beispiel 35*

*9-Amino-5-oxo-6-benzyl-2,3,5,6-tetrahydroimidazo[1,2-c]chinazolin*

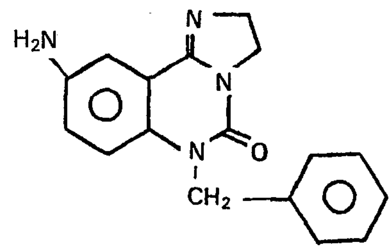

Fp.: 195-198°C;
Ausbeute: 20%

*Beispiel 36*

*9-Amino-5-oxo-6-methyl-2,3,5,6-tetrahydroimidazo[1,2-c]chinazolin*

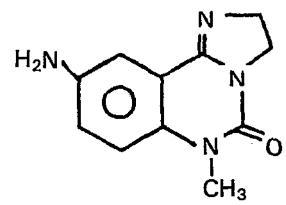

Fp.: 273-275°C;
Ausbeute: 11%

*Beispiel 37*

*9-Isovaleroylamino-5-oxo-2,3,5,6-tetrahydroimidazo[1,2-c]chinazolin*

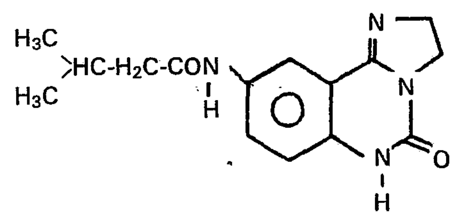

Zu 2,02 g (0,01 Mol) 9-Amino-5-oxo-2,3,5,6-tetrahydroimidazo[1,2-c]chinazolin in 30 ml absolutem Pyridin gibt man 2,5 g (0,02 Mol) Isovaleriansäurechlorid und rührt 8 Stunden bei Raumtemperatur. Anschliessend verdünnt man mit Ether, trennt die ausgefallenen Kristalle ab. Die Kristalle werden in Wasser gelöst, die wässrige Lösung wird mit Aktivkohle gereinigt und anschliessend die freie Base durch Zugabe von Ammoniak gefällt. Es wird abgesaugt und getrocknet.

Ausbeute: 1,4 g (49% der Theorie);
Fp.: > 305°C.

Analog Beispiel 37 wurden hergestellt:

*Beispiel 38*

*9-Acetamido-5-oxo-2,3,5,6-tetrahydroimidazo-[1,2-c]chinazolin*

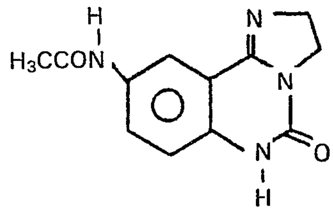

Fp.: > 300°C;
Ausbeute: 49%

*Beispiel 39*
*9-Propionylamino-5-oxo-2,3,5,6-tetrahydroimidazo[1,2-c]chinazolin*

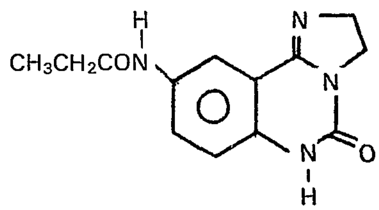

Fp.: > 305°C;
Ausbeute: 50%

*Beispiel 40*
*9-n-Valeroylamino-5-oxo-2,3,5,6-tetrahydroimidazo[1,2-c]chinazolin*

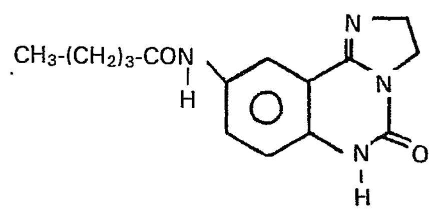

Fp.: 298-300°C;
Ausbeute: 35%

*Beispiel 41*
*9-Nonanoylamino-5-oxo-2,3,5,6-tetrahydroimidazo[1,2-c]chinazolin*

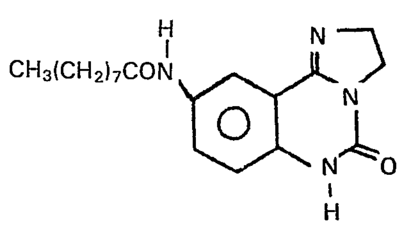

Fp.: 268-270°C;
Ausbeute: 93%

*Beispiel 42*
*9-Acetylamino-5-oxo-6-allyl-2,3,5,6-tetrahydroimidazo[1,2-c]chinazolin*

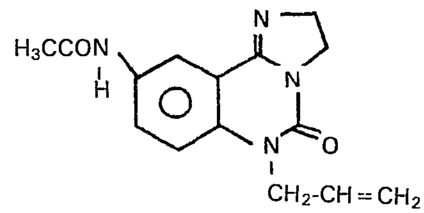

Fp.: 232-234°C;
Ausbeute: 32%

*Beispiel 43*
*9-Propionylamino-5-oxo-6-allyl-2,3,5,6-tetrahydroimidazo[1,2-c]chinazolin*

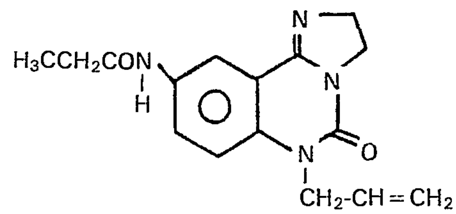

Fp.: 149-152°C;
Ausbeute: 63%

*Beispiel 44*
*9-Isovaleroylamino-5-oxo-6-allyl-2,3,5,6-tetrahydroimidazo[1,2-c]chinazolin*

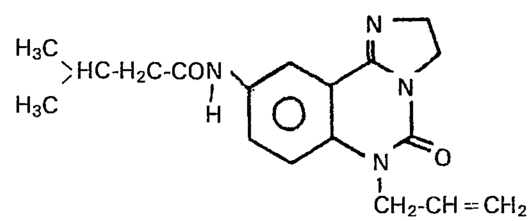

Fp.: 202-205°C;
Ausbeute: 73%

Analog Beispiel 37 wurden mit den entsprechenden Chlorkohlensäureestern hergestellt:

*Beispiel 45*
*9-Methoxycarbonylamino-5-oxo-2,3,5,6-tetrahydroimidazo[1,2-c]chinazolin*

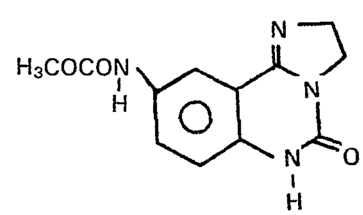

Fp.: > 300°C;
Ausbeute: 97%

*Beispiel 46*
*9-Ethoxycarbonylamino-5-oxo-2,3,5,6-tetrahydroimidazo[1,2-c]chinazolin*

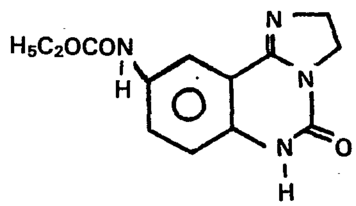

Fp.: 300°C;
Ausbeute: 98%

*Beispiel 47*

*9-i-Butyloxycarbonylamino-5-oxo-2,3,5,6-tetrahydroimidazo[1,2-c]chinazolin*

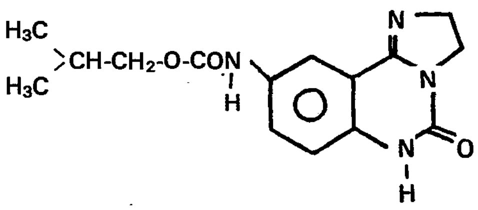

Fp.: 300°C;
Ausbeute: 43%

*Beispiel 48*

*9-N'-Methylureido-5-oxo-2,3,5,6-tetrahydroimidazo[1,2-c]chinazolin*

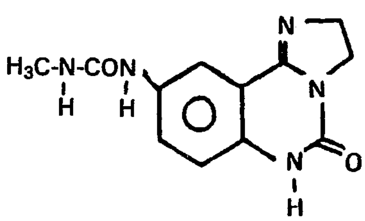

Zu 2,02 g (0,01 Mol) 9-Amino-5-oxo-2,3,5,6-tetrahydroimidazo[1,2-c]chinazolin und 2 Tropfen Sn-Octoat in 30 ml Pyridin gibt man bei Raumtemperatur 5 ml Methylisocyanat. Dann wird 1 Stunde auf 80°C erhitzt. Nach dem Abkühlen wird das Reaktionsprodukt mit Ether aus der Lösung gefällt und abgesaugt. Es wird mit Wasser ausgerührt, erneut abgesaugt und getrocknet.

Ausbeute: 1,7 g (66% der Theorie);
Fp.: > 300°C.

Analog Beispiel 48 wurden hergestellt:

*Beispiel 49*

*9-N'-n-Propylureido-5-oxo-2,3,5,6-tetrahydroimidazo[1,2-c]chinazolin*

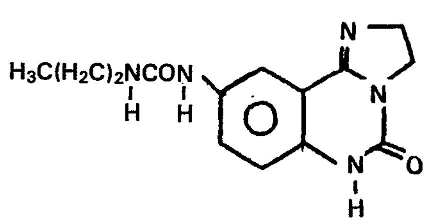

Fp.: > 300°C;
Ausbeute: 59%

*Beispiel 50*

*9-N'-Phenylureido-5-oxo-2,3,5,6-tetrahydroimidazo[1,2-c]chinazolin*

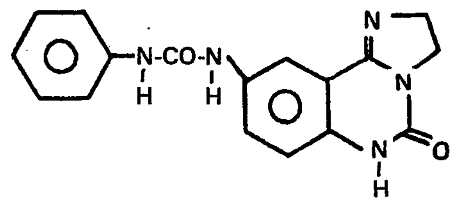

Fp.: > 300°C;
Ausbeute: 78%

*Beispiel 51*

*9-N'-Benzoylureido-5-oxo--2,3,5,6-tetrahydroimidazo[1,2-c]chinazolin*

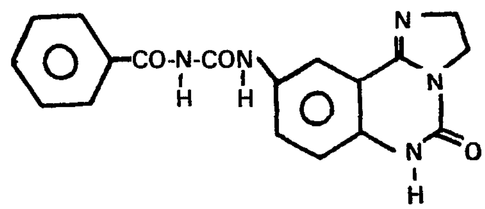

Fp.: > 305°C;
Ausbeute: 99%

Mit Isothiocyanaten als Ausgangsstoffen wurden analog Beispiel 48 erhalten:

*Beispiel 52*

*9-N'-Methylthioureido-5-oxo-2,3,5,6-tetrahydroimidazo[1,2-c]chinazolin*

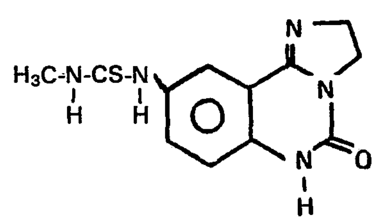

Fp.: > 300°C;
Ausbeute: 69%

*Beispiel 53*

*9-N'-Phenylthioureido-5-oxo-2,3,5,6-tetrahydroimidazo[1,2-c]chinazolin*

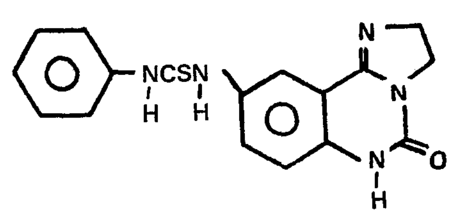

Fp.: > 300°C;
Ausbeute: 56%

*Beispiel 54*

*9-(4-Morpholinylsulfonyl)-5-oxo-2,3,5,6-tetrahydroimidazo[1,2-c]chinazolin*

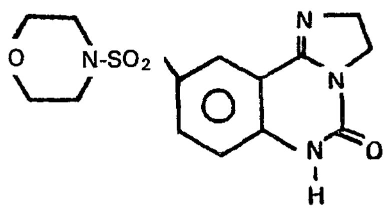

Zu 20 ml Chlorsulfonsäure werden unter Eiskühlung 30 mmol 5-Oxo-2,3,5,6-tetrahydroimidazo-[1,2-c]chinazolin gegeben und 3 Stunden bei 50 bis 60°C gerührt. Nach Abkühlung wurde diese Lösung vorsichtig zu 80 ml gekühltem Morpholin getropft und danach mit 150 ml Wasser verdünnt. Es wurde abgesaugt, mit Wasser gewaschen und mit Alkohol ausgekocht.

Fp.: > 260°C;
Ausbeute: 27%

*Beispiel 55*

*9-Aminosulfonyl-5-oxo-2,3,5,6-tetrahydroimidazo-[1,2-c]chinazolin*

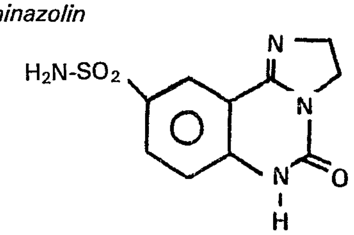

Fp.: > 300°C;
Ausbeute: 23%
(Herstellung analog Beispiel 54 durch Eintropfen in konz. $NH_3$).

*Beispiel 56*

*9-(1-Piperazinylsulfonyl)-5-oxo-2,3,5,6-tetrahydroimidazo[1,2-c]chinazolin*

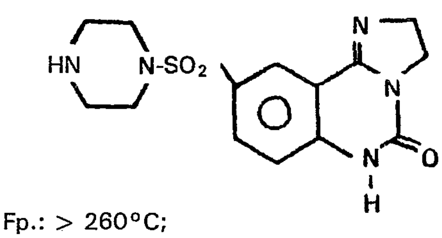

Fp.: > 260°C;
Ausbeute: 18%
(Herstellung analog Beispiel 54 durch Eintropfen in Piperazin).

*Beispiel 57*

*9-Hydroxysulfonyl-5-oxo-2,3,5,6-tetrahydroimidazo[1,2-c]chinazolin*

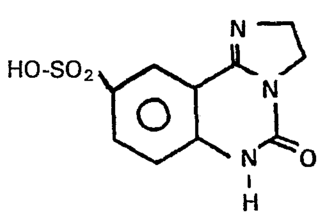

Fp.: > 300°C;
(Herstellung analog Beispiel 54 durch Eintropfen in Wasser).

*Beispiel 58*

*5-Oxo-2-methyl-2,3,5,6-tetrahydroimidazo[1,2-c]-chinazolin*

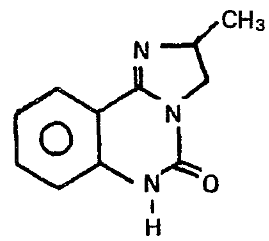

Darstellung analog Beispiel 1 durch Umsetzen mit 1,2-Diaminopropan statt Ethylendiamin.
Schmp. 255-258°C;
Ausbeute: 20%

*Beispiel 59*

*5-Oxo-(2- und 3-phenyl)-2,3,5,6-tetrahydroimidazo[1,2-c]chinazolin-hydrochlorid*

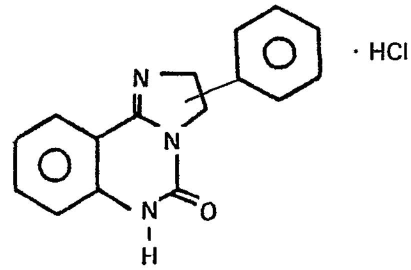

2:3-Isomerengemisch

Analog Beispiel 1 durch Umsetzen mit 2 eq. Phenyl-1,2-diaminoethan.

Nach Erhitzen auf 200°C in Tetralin und Abziehen des Tetralins im Vakuum wird der Rückstand in Pyridin gelöst und bei Raumtemperatur mit einem Äquivalent Chlorameisensäurephenylester versetzt.

Nach Rühren über Nacht wird mit Wasser versetzt, einrotiert und zusammen mit Tetralin 1 Stunde auf 220°C erhitzt. Nach Anziehen des Tetralins im Vakuum wird mit Ethanol versetzt und über Nacht kristallisiert.

Schmp. 246-248°C;
Ausbeute: 10%

*Beispiel 60*

*6-Oxo-2,3,5,6,7-hexahydropyrimido[1,2-c]-chinazolin*

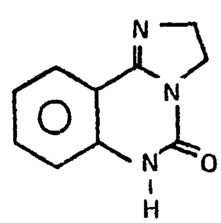

Darstellung analog Beispiel 1 mit 1,3-Diaminopropan anstelle von Ethylendiamin.
Fp.: 248-250°C (aus Isopropanol);
Ausbeute: 35%

*Beispiel 61*

*9-Benzolsulfonylamino-5-oxo-2,3,5,6-tetrahydro-imidazo[1,2-c]chinazolin*

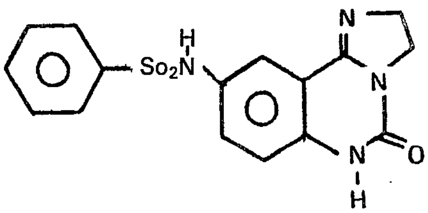

Zu 4,04 g 9-Amino-5-oxo-2,3,5,6-tetrahydro-imidazo[1,2-c]chinazolin in 50 ml abs. Pyridin wurden unter Eiskühlung 7,1 g Benzolsulfonsäurechlorid gegeben. Die Kühlung wurde entfernt und es wurde 2 Stunden bei Raumtemperatur gerührt. Dann wurde das Reaktionsgemisch mit viel $H_2O$ versetzt und die ausgefallenen Kristalle wurden abgesaugt. Die Kristalle wurden in wässrigem $NH_3$ gelöst, die Lösung wurde mit Aktivkohle geklärt und mit Salzsäure neutralisiert.

Die ausgefallenen Kristalle wurde abgesaugt, mit Ethanol und Ether gewaschen und getrocknet.

Fp.: > 300°C;

Ausbeute: 4,4 g (64%)

Analog zu Beispiel 61 wurden hergestellt:

*Beispiel 62*

*9-o-Toluolsulfonylamino-5-oxo-2,3,5,6-tetrahydro-imidazo[1,2-c]chinazolin*

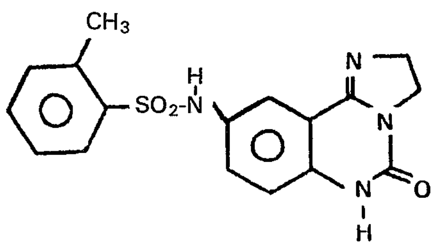

Fp.: > 300°C;

Ausbeute: 72%

*Beispiel 63*

*9-p-Nitrobenzolsulfonylamino-5-oxo-2,3,5,6-tetra-hydroimidazo[1,2-c]chinazolin*

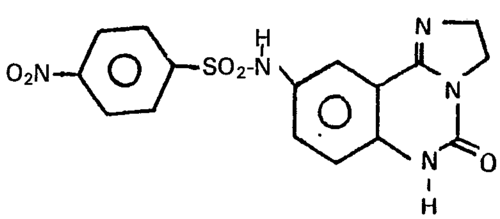

Fp.: > 300°C;

Ausbeute: 36%

*Beispiel 64*

*9-Methansulfonylamino-5-oxo-2,3,5,6-tetrahydro-imidazo[1,2-c]chinazolin*

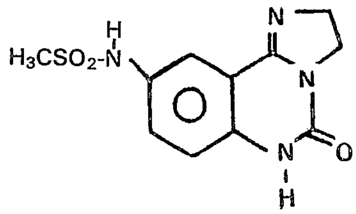

Fp.: > 300°C;

Ausbeute: 59%

*Beispiel 65*

*9-Benzoylamino-5-oxo-2,3,5,6-tetrahydroimidazo-2-c]chinazolin*

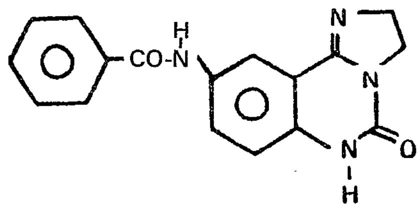

Fp.: > 305°C (aus Dimethylformamid);

Ausbeute: 58%.

Die Verbindung wurde in Analogie zu Beispiel 37 hergestellt.

*Beispiel 66*

*9-Nitro-5-oxo-(2- oder 3-methyl)-2,3,5,6-tetra-hydroimidazo[1,2-c]chinazolin*

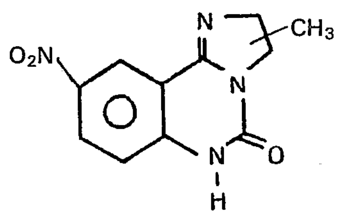

Ausbeute: 52%,

Fp.: 265-8°C.

Darstellung analog Beispiel 31 durch Nitrierung von 5-Oxo-(2- oder 3-methyl)-2,3,5,6-tetrahydro-imidazo[1,2-c]chinazolin.

*Beispiel 67*

*9-Trifluormethyl-5-oxo-(2- oder 3-methyl)-2,3,5,6--tetrahydroimidazo[1,2-c]chinazolin*

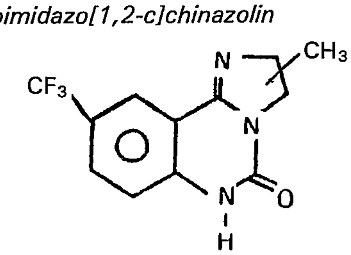

Ausbeute: 5%,

Fp.: 270-3°C.

Darstellung analog Beispiel 1.

*Beispiel 68*

*9-Trifluormethyl-5-oxo-(3- oder 2-methyl)-2,3,5,6-tetrahydroimidazo[1,2-c]chinazolin*

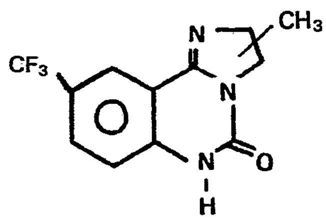

Ausbeute: 3%,
Fp.: 190-5°C.

Isolierung aus der Mutterlauge des Beispieles 67 durch Chromatographie mit Methanol.

*Beispiel 69*
*9-Amino-5-oxo-(2- oder 3-methyl)--2,3,5,6-tetrahydroimidazo[1,2-c]chinazolin*

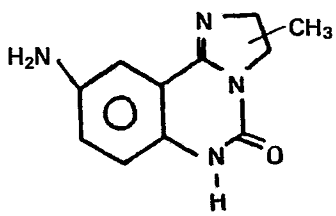

Ausbeute: 40%,
Fp.: > 260°C.
Darstellung analog Beispiel 33.

*Beispiel 70*
*9-(4-Morpholinylsulfonyl)-5-oxo-(2- oder 3-methyl)-2,3,5,6-tetrahydroimidazo[1,2-c]chinazolin*

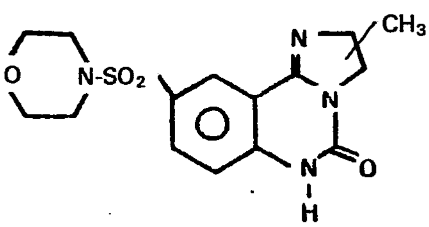

Ausbeute: 33%,
Fp.: > 260°C.
Darstellung analog Beispiel 54.

*Beispiel 71*
*9-Isovaleroylamino-5-oxo-(2- oder 3-methyl)-2,3,5,6-tetrahydroimidazo[1,2-c]chinazolin*

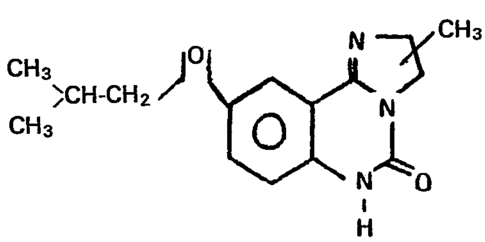

Ausbeute: 27%,
Fp.: > 260°C.
Darstellung analog Beispiel 37.

*Beispiel 72*
*9-(2-Hydroxyethyl)-aminosulfonyl-5-oxo-(2- oder 3-methyl)-2,3,5,6-tetrahydroimidazo[1,2-c]-chinazolin*

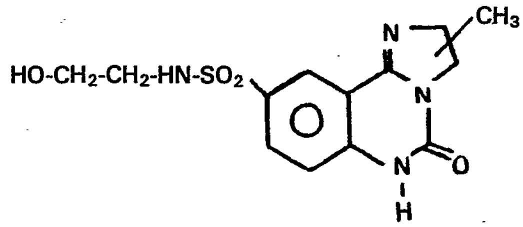

Ausbeute: 5%,
Fp.: > 241-50°C.
Darstellung analog Beispiel 54 durch Eintropfen in 2-Aminoethanol.

*Beispiel 73*
*9-Trifluormethyl-5-oxo-(2- oder 3-Methyl)-6-allyl-2,3,5,6-tetrahydroimidazo[1,2-c]chinazolin*

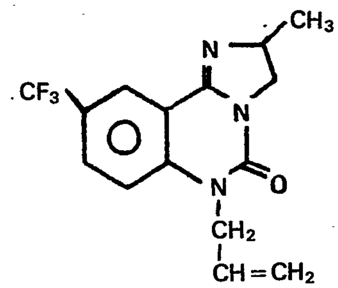

Ausbeute: 68%,
Fp.: > 94-96°C.
Darstellung analog Beispiel 29.

*Beispiel 74*
*9-Trifluormethoxy-6-oxo-dimethyl-2,3,4,5,6,7-hexahydropyrimido[1,2-c]chinazolin*

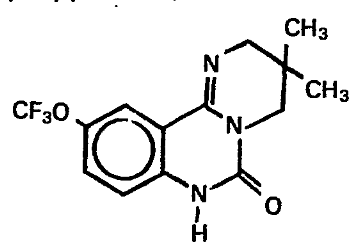

Ausbeute: 11%,
Fp.: > 210-215°C.
Darstellung analog Beispiel 1 mit 1,3-Diamino-2,2-dimethylpropan.

*Beispiel 75*
*9-Trifluormethyl-6-oxo-3,3-dimethyl-2,3,4,5,6,7-hexahydropyrimido[1,2-c]chinazolin*

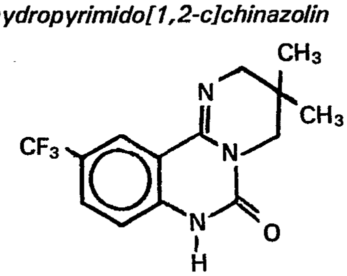

Ausbeute: 11%,
Fp.: > 260°C.
Darstellung analog Beispiel 1 mit 1,3-Diamino-2,2-dimethylpropan.

*Beispiel 76*
*9-Trifluormethoxy-5-oxo-(3- oder 2-methyl)-2,3,5,6-tetrahydroimidazo[1,2-c]chinazolin*

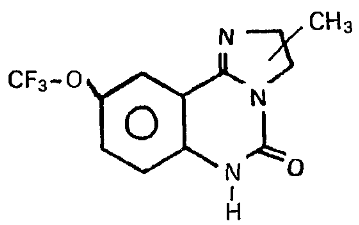

*Ausbeute: 10%,*
*Fp.:* > 240-2°C.
Darstellung analog Beispiel 1 und Ausfällung mit Aceton.

*Beispiel 77*
*9-Trifluormethoxy-5-oxo(2- oder 3-methyl)-2,3,5,6-tetrahydroimidazo[1,2-c]chinazolin*

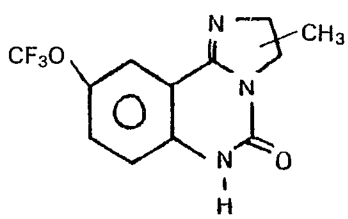

Ausbeute: 15%,
Fp.: > 205-210°C.
Isolierung aus der Mutterlauge von Beispiel 76 durch Chromatographie mit Essigester.

*Beispiel 78*
*7,9-Dibrom-5-oxo-(2- und 3-methyl)-2,3,5,6-tetrahydroimidazo[1,2-c]chinazolin*

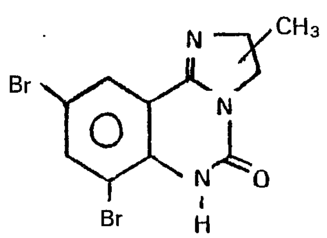

1:1-Isomerengemisch

Ausbeute: 30%,
Fp.: > 220-5°C.
Darstellung analog Beispiel 1.

*Beispiel 79*
*7,9-Dichlor-5-oxo-(2- und 3-methyl)-2,3,5,6-tetrahydroimidazo[1,2-c]chinazolin*

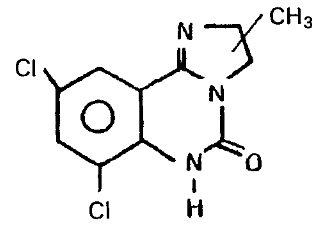

Isomerengemisch

Ausbeute: 21%,
Fp.: > 215-9°C.
Darstellung analog Beispiel 1.

*Beispiel 80*
*6-Oxo-3-hydroxy-2,3,4,5,6,7-hexahydropyrimido-[1,2-c]chinazolin*

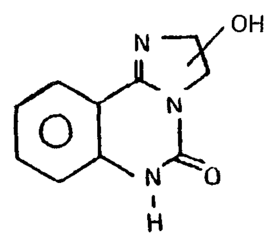

Ausbeute: 25%,
Fp.: > 260°C.
Darstellung analog Beispiel 1 mit 1,3-Diaminopropanol-2 anstelle von Ethylendiamin.

*Beispiel 81*
*10-Nitro-6-oxo-2,3,4,5,6,7-hexahydropyrimido-[1,2-c]chinazolin*

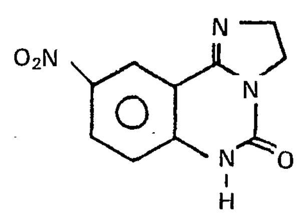

Darstellung analog Beispiel 31 durch Nitrierung von 6-Oxo-2,3,4,5,6,7-hexahydropyrimido[1,2-c]-chinazolin.
Fp.: > 300°C;
Ausbeute: 54,8%.

*Beispiel 82*
*10-Amino-6-oxo-2,3,4,5,6,7-hexahydropyrimido-[1,2-c]chinazolin*

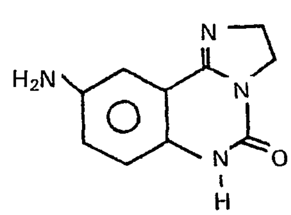

Darstellung analog Beispiel 33 durch Reduktion von 10-Nitro-6-oxo-2,3,4,5,6,7-hexahydropyrimido[1,2-c]chinazolin mit Natriumdithionit.
Fp.: 280°C (Zers.),
Ausbeute: 50%.

*Beispiel 83*

*10-Nitro-6-oxo-7-allyl-2,3,4,5,6,7-hexahydropyrimido[1,2-c]chinazolin*

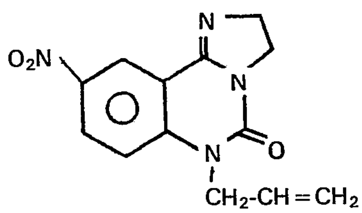

Darstellung analog Beispiel 29 aus 10-Nitro-6-oxo-2,3,4,5,6,7-hexahydropyrimido[1,2-c]chinazolin und Allylbromid.
Fp.: 242-245°C,
Ausbeute: 45,5%.

*Beispiel 84*

*9-(3-Methylbenzoylamino)-5-oxo--2,3,5,6-tetrahydroimidazo[1,2-c]chinazolin*

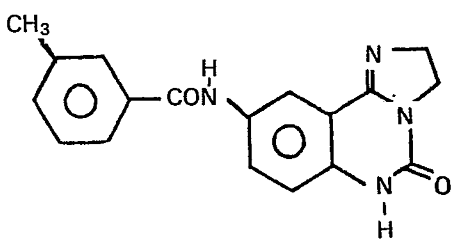

Darstellung analog Beispiel 37.
Fp.: > 300°C;
Ausbeute: 39%.

*Beispiel 85*

*9-(4-Chlorbenzoylamino)-5-oxo-2,3,5,6-tetrahydroimidazo[1,2-c]chinazolin*

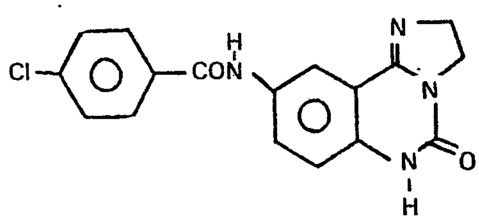

Darstellung analog Beispiel 37.
Fp.: > 300°C;
Ausbeute: 38%.

*Beispiel 86*

*9-(2,5-Dichlorbenzoylamino)-5-oxo-2,3,5,6-tetrahydroimidazo[1,2-c]chinazolin*

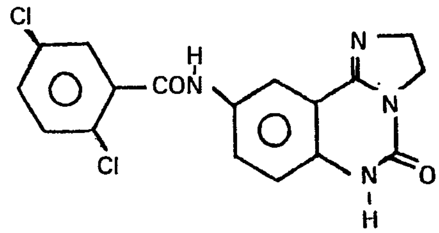

Darstellung analog Beispiel 37.
Fp.: > 300°C;
Ausbeute: 27%.

*Beispiel 87*

*9-(4-Chlor-3-nitrobenzoylamino)-5-oxo-2,3,5,6-tetrahydroimidazo[1,2-c]chinazolin.*

Darstellung analog Beispiel 37.
Fp.: > 300°C;
Ausbeute: 70%.

*Beispiel 88*

*10-Trifluormethyl-6-oxo-2,3,4,5,6,7-hexahydropyrimido[1,2-c]chinazolin*

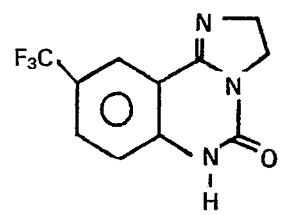

Darstellung analog Beispiel 1 aus 2-Amino-5-trifluormethylbenzoesäure und 1,3-Diaminpropan.
Fp.: 259-260°C;
Ausbeute: 35%.

*Beispiel 89*

*10-Trifluormethyl-6-oxo-7-allyl-2,3,4,5,6,7-hexahydropyrimido[1,2-c]chinazolin*

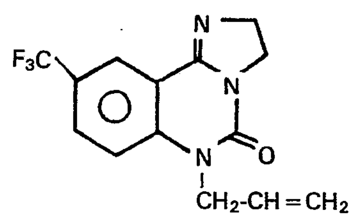

Darstellung analog Beispiel 29 aus 10-Trifluormethyl-6-oxo-2,3,4,5,6,7-hexahydropyrimido-[1,2-c]chinazolin und Allylbromid.
Fp.: 122-125°C;
Ausbeute: 30%.

*Beispiel 90*

*10-Trifluormethoxy-6-oxo-2,3,4,5,6,7-hexahydropyrimido[1,2-c]chinazolin*

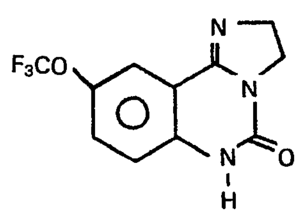

Darstellung analog Beispiel 1 aus 2-Amino-5-trifluormethoxybenzoesäure und 1,3-Diaminopropren.
Fp.: 257-260°C,
Ausbeute: 28%.

*Beispiel 91*

*10-Trifluormethoxy-7-allyl-6-oxo-2,3,4,5,6,7--hexahydropyrimido[1,2-c]chinazolin*

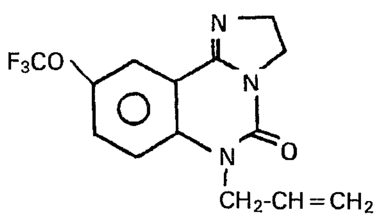

Darstellung analog Beispiel 29 aus 10-Trifluormethoxy-6-oxo-2,3,4,5,6,7-hexahydropyrimido-[1,2-c]chinazolin und Allylbromid.
Fp.: 103-105°C,
Ausbeute: 37%.

*Beispiel 92*

*5-Oxo-(2,2- oder 3,3)-dimethyl-2,3,5,6-tetrahydroimidazo[1,2-c]chinazolin*

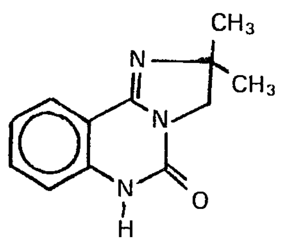

oder

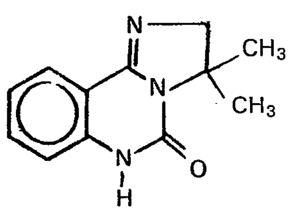

Struktur nicht geklärt.
Darstellung analog Beispiel 1 aus Anthranilsäure und 1,2-Diamino-2-methyl-propan.
Fp.: 253-255°C,
Ausbeute: 32%.

*Beispiel 93*

*9-Trifluormethyl-5-oxo-(2,2- oder 3,3)-dimethyl--2,3,5,6-tetrahydroimidazo[1,2-c]chinazolin*

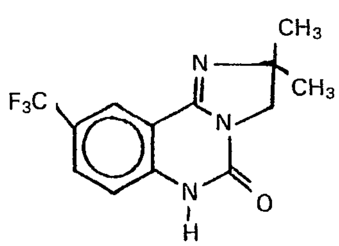

oder

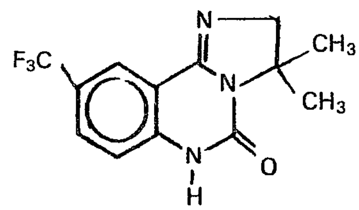

Struktur nicht geklärt.
Darstellung analog Beispiel 1 aus 2-Amino-5-trifluormethylbenzoesäure und 1,2-Diamino-2-methyl--propan.
Fp.: 228-232°C,
Ausbeute: 25%.

*Beispiel 94*

*9-Phenylacetamido-5-oxo-2,3,5,6-tetrahydroimidazo[1,2-c]chinazolin*

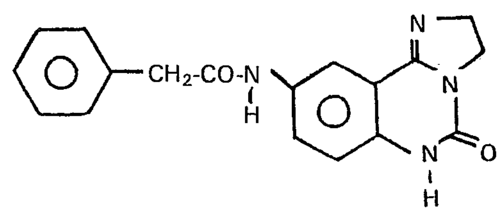

Darstellung analog Beispiel 37.
Fp.: >300°C,
Ausbeute: 78%.

*Beispiel 95*

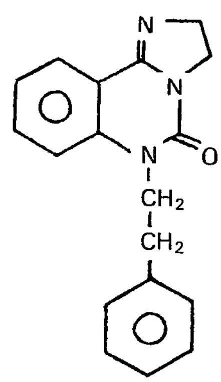

Darstellung analog Beispiel 29 aus 5-Oxo-2,3,5,6--tetrahydroimidazo[1,2-c]chinazolin und 2-Phenylethylbromid.
Fp.: 133-135°C,
Ausbeute: 48,1%.

*Beispiel 96*

*9-Acetamido-5-oxo-6-methyl-2,3,5,6-tetrahydroimidazo[1,2-c]chinazolin*

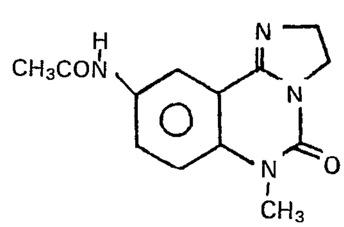

Darstellung analog Beispiel 37 aus 9-amino-5--oxo-2,3,5,6-tetrahydroimidazo[1,2-c]chinazolin und Acetanhydrid.
Fp.: 283-286°C,
Ausbeute: 70%.

*Beispiel 97*
*9-Propionylamino-5-oxo-6-methyl-2,3,5,6-tetrahydroimidazo[1,2-c]chinazolin*

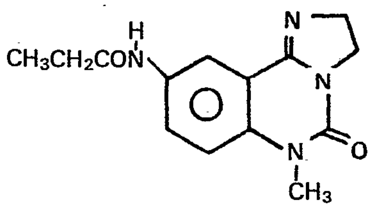

Darstellung analog Beispiel 37 aus 9-Amino-5--oxo-2,3,5,6-tetrahydroimidazo[1,2-c]chinazolin und Propionsäurechlorid.
Fp.: 215-218°C,
Ausbeute: 59%.

*Beispiel 98*
*9-Isovaleroylamino-5-oxo-6-methyl-2,3,5,6-tetrahydroimidazo[1,2-c]chinazolin*

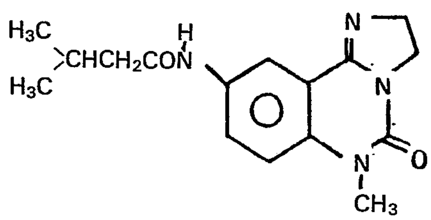

Darstellung analog Beispiel 37 aus 9-Amino-5--oxo-2,3,5,6-tetrahydroimidazo[1,2-c]chinazolin und Isovaleriansäurechlorid.
Fp.: 137-140°C,
Ausbeute: 60%.

*Beispiel 99*
*9-Acetoamido-5-oxo-6-benzyl-2,3,5,6-tetrahydroimidazo[1,2-c]chinazolin*

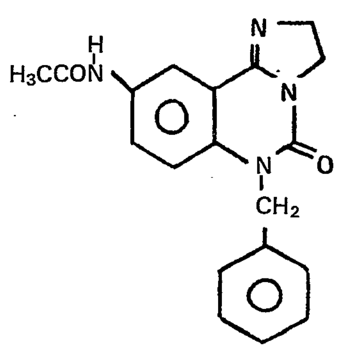

Darstellung analog Beispiel 37 aus 9-Amino-5--oxo-6-benzyl--2,3,5,6-tetrahydroimidazo[1,2-c]-chinazolin und Acetanhydrid.
Fp.: 270-272°C,
Ausbeute: 84%.

*Beispiel 100*
*9-Propionylamino-5-oxo-6-benzyl-2,3,5,6-tetrahydroimidazo[1,2-c]chinazolin*

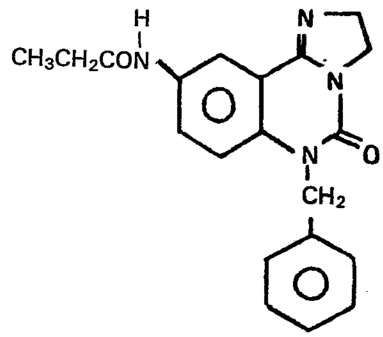

Darstellung analog Beispiel 37 aus 9-Amino-5--oxo-6-benzyl-2,3,5,6-tetrahydroimidazo[1,2-c]-chinazolin und Propionsäurechlorid.
Fp.: 125-129°C,
Ausbeute: 75%.

*Beispiel 101*
*9-Isovaleroylamino-5-oxo-6-benzyl-2,3,5,6-tetrahydroimidazo[1,2-c]chinazolin*

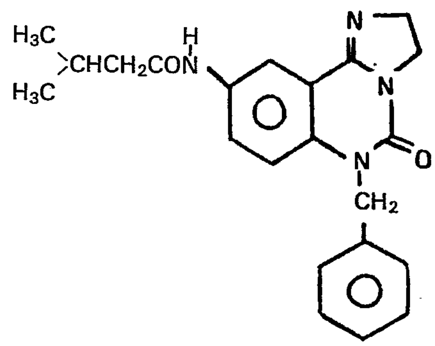

Darstellung analog Beispiel 37 aus 9-Amino-5--oxo-6-benzyl-2,3,5,6-tetrahydroimidazo[1,2-c]-chinazolin und Isovaleriansäurechlorid.
Fp.: 173-178°C,
Ausbeute: 95%.

*Beispiel 102*
*6-(3-Trifluormethylphenyl)-2,3-dihydro-imidazol-[1,2-c]chinazolin-5-on*

a) *2-[2-(3-Trifluormethylphenylamino)-phenyl]--imidazolin-2*

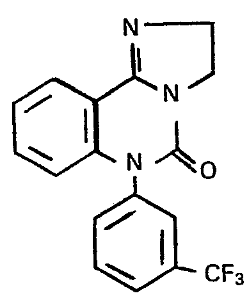

Ein Gemisch aus 20,0 g (60 mMol) 2-(3-Trifluormethylphenylamino)-benzoesäure-n-butylester und

28,2 ml (0,42 Mol) Ethylendiamin wird 21 h im Autoklaven auf 200°C erhitzt. Nach Abkühlung wird das Reaktionsgemisch mit Wasser verdünnt und mit Dichlormethan extrahiert. Der Extrakt wird über $Na_2SO_4$ getrocknet, eingedampft und an Kieselgel chromatographiert (Laufmittel Essigester/Methanol 7:3).

Ausbeute 30% der Theorie
Fp.: 95-96°C (Schaum).

b) 6-(3-Trifluormethylphenyl)-2,3-dihydro-imidazo[1,2-c]chinazolin-5-on

Eine Lösung von 10,4 g (34 mMol) 2-[2-(3-Trifluormethylphenylamino)-phenyl]-imidazolin-2 in 100 ml abs. Tetrahydrofuran wird mit 3,26 g (75 mMol) Natriumhydrid versetzt und bis zur Beendigung der Wasserstoffentwicklung auf 50-60°C erwärmt (5-6 h). Nach Abkühlung auf Raumtemperatur werden 16 ml (0,166 Mol) Chlorameisensäureethylester zugetropft und das Reaktionsgemisch eine Stunde unter Rückfluss gekocht. Man lässt abkühlen, stellt den pH-Wert des Ansatzes mit Eisessig auf 4-5 ein, verdünnt mit Wasser und extrahiert mit Dichlormethyl. Die organische Phase wird über $Na_2SO_4$ getrocknet, eingedampft, der Rückstand an Kieselgel chromatographiert (Laufmittel Eisessig/Methanol 7:3) und der Verdampfungsrückstand der produkthaltigen Fraktionen mit Ether/Petrolether 1:3 ausgerührt.

Ausbeute 24% der Theorie
Fp.: 211-213°C.

*Beispiel 103*

*6-(2-Fluor-4-nitrophenyl)-2,3-dihydro-imidazo-[1,2-c]chinazolin-5-on*

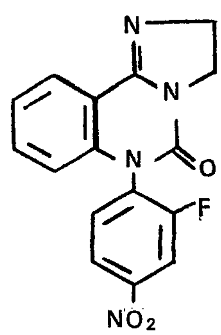

a) *2-(2-Fluor-4-nitrophenylamino)-benzonitril*

Eine Lösung von 59 g (0,5 Mol) 2-Aminobenzonitril und 53,2 ml (0,5 Mol) 3-Fluornitrobenzol in abs. Dimethylformamid wird portionsweise mit 21,8 g (0,5 Mol) Natriumhydrid versetzt, wobei sich das Reaktionsgemisch unter Verfärbung von dunkelgrün nach dunkelrot auf 80°C erwärmt. Nach Abkühlung auf Raumtemperatur wird mit Wasser verdünnt, mit 2n Salzsäure angesäuert und der entstehende Niederschlag abfiltriert. Zur Reinigung wird der Niederschlag an Kieselgel mit Dichlormethan chromatographiert und der Verdampfungsrückstand der produkthaltigen Fraktionen mit Ether/Petrolether 1:2 ausgerührt.

Ausbeute 27% der Theorie
Fp.: 185-186°C.

b) *2-[2-(Fluor-4-nitrophenylamino)-phenyl]-imidazolin-2*

Ein Gemisch aus 8,5 g (33 mMol) 2-(2-Fluor-4-nitrophenylamino)-benzonitril und 16,7 ml (249 mMol) Ethylendiamin wird im Autoklav 1h auf 180° erhitzt und das Reaktionsgemisch anschliessend wie unter Beispiel 1a) beschrieben, aufgearbeitet. Nach Kristallisation aus Dichlormethan und Ether/Petrolether wird so das Produkt mit 60% Ausbeute erhalten.

Fp.: 155-156°C.

c) *6-(2-Fluor-4-nitrophenyl)-2,3-dihydro-imidazo[1,2-c]chinazolin-5-on*

Eine Lösung von 3,47 g (11,6 mMol) 2-[2-(2-Fluor-4-nitrophenylamino)-phenyl]-imidazolin-2 in 60 ml abs. Tetrahydrofuran wird mit 1,1 g (25 mMol) Natriumhydrid versetzt und bis zur Beendigung der Wasserstoffentwicklung auf 50°C erwärmt. Nach Abkühlung auf Raumtemperatur werden 5,8 ml (60 mMol) Chlorameisensäureethylester in 10 ml abs. Tetrahydrofuran zugetropft und 90 min auf 50°C erwärmt. Man lässt auf Raumtemperatur erkalten, säuert das Reaktionsgemisch mit Eisessig an, verdünnt es mit Wasser und extrahiert mit Dichlormethan. Die organische Phase wird eingedampft, der Rückstand mit Wasser versetzt, die Lösung mit Salzsäure auf einen pH-Wert von 1-2 eingestellt und mit Dichlormethan extrahiert. Der beim Versetzen der wässrigen Phase mit $KHCO_3$-Lösung auftretende Niederschlag wird abgesaugt, mit Wasser gewaschen und getrocknet.

Ausbeute 27% der Theorie
Fp. 223-224°C.

*Beispiel 104*

*6-(3-Nitrophenyl)-2,3-dihydro-imidazo[1,2-c]chinazolin-5-on*

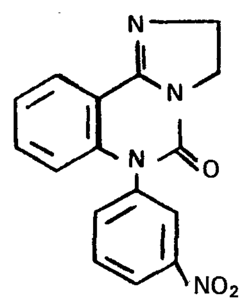

a) *2-(3-Nitrophenylamino)-benzonitril*

3,85 g (15 mMol) 2-(3-Nitrophenylamino)-benzoesäureamid werden mit 30 ml (0,325 Mol) Phosphoroxychlorid übergossen und anschliessend tropfenweise (Erwärmung) mit 4,32 ml (31 mMol) Triethylamin versetzt. Nach 30 min wird überschüssiges Phosphoroxychlorid abdestilliert, der Rückstand mit Wasser ausgerührt und das Produkt abgesaugt.

Ausbeute: 3,2 g (89,3% der Theorie)
Fp.: 165-166°C.

b) *2-[2-(3-Nitrophenylamino)-phenyl]-imidazolin-2*

Ein Gemisch aus 2,39 g (10 mMol) 2-(3-Nitrophenylamino)-benzonitril und 4,7 ml (70 mMol) Ethylendiamin wird im Autoklav 2 1/2h auf 180°C erhitzt und das Reaktionsgemisch, wie unter Beispiel 2b) beschrieben, aufgearbeitet.

Ausbeute: 1 g (35,5% der Theorie)

Fp.: 113-115°C.

c) *6-(3-Nitrophenyl)-2,3-dihydro-imidazo[1,2-c]-chinazolin-5-on*

1,21 g (4,3 mMol) 2-[2-(3-Nitrophenylamino)--phenyl]-imidazolin-2 werden in 10 ml Toluol suspendiert, mit 2,4 ml (17 mMol) Triethylamin und anschliessend mit 10 ml 10proz. Lösung von Phosgen in Toluol versetzt. Der sich unter Erwärmen bildende Brei wird durch Zugabe von 85 ml Dichlormethan in Lösung gebracht und das Reaktionsgemisch 1h unter Rückfluss gekocht. Nach Abkühlung wird der Ansatz mit $KHCO_3$-Lösung und Wasser gewaschen, die organische Phase nach Trocknen über $Na_2SO_4$ eingedampft und der Rückstand an Kieselgel chromatographiert. (Laufmittel Essigester/Methanol 7:3). Die produkthaltigen Fraktionen werden eingedampft, der Rückstand in 2n Salzsäure aufgenommen, die Lösung mit Aktivkohle behandelt und nach Filtration mit 2n Natronlauge auf einen pH-Wert von 9-10 gestellt. Der auftretende Niederschlag wird abgesaugt, mit Wasser gewaschen und getrocknet.

Ausbeute: 350 mg (26,3% der Theorie)

Fp.: 270-271°C.

**Patentansprüche**

1. Tricyclische Cytosinderivate der allgemeinen Formel (I)

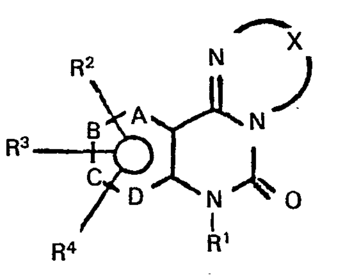

(I)

in welcher

X für ein gesättigtes Brückenglied mit 2 oder 3 Kohlenstoffatomen steht, welches gegebenenfalls durch 1 oder 2 Reste aus der Reihe Alkyl mit 1 bis 4 Kohlenstoffatomen, Phenyl oder Benzyl substituiert ist,

A, B, C und D jeweils für CH stehen, wobei gegebenenfalls 1 oder 2 dieser CH-Gruppen durch N ersetzt sind,

$R^1$ für Wasserstoff, Alkyl, Alkenyl oder Alkinyl mit jeweils bis zu 18 Kohlenstoffatomen steht, wobei die Alkenyl- und Alkinylreste 1, 2 oder 3 Doppel- oder Dreifachbindungen enthalten können, oder für einen mono-, bi- oder tricyclischen aliphatischen Rest mit 3 bis 8 Kohlenstoffatomen, der gesättigt oder einfach oder doppelt ungesättigt sein kann oder für einen gegebenenfalls durch Trifluormethyl, Fluor und Nitro oder Nitro substituierten Phenylrest steht, wobei die genannten Alkyl- und Alkenylreste substituiert sein können durch Substituenten aus der folgenden Gruppe: Hydroxy, Alkoxy mit 1 bis 4 Kohlenstoffatomen, Phenoxy, wobei der Phenylrest durch $NO_2$, Amino, Acet-amido, $C_1$-$C_4$- Alkyl, $C_1$-$C_4$-Alkoxy, Halogen $CF_3$, Carboxy oder Carbalkoxy mit 1 bis 4 Kohlenstoffatomen im Alkoxyrest substituiert sein kann; Acyloxy, wobei der Acylrest von aliphatischen Carbonsäuren mit 1 bis 7 C-Atomen oder Phenylcarbonsäuren, die im Phenylrest durch OH, Halogen, $C_1$-$C_4$-Alkyl, $C_1$-$C_4$-Alkoxy, Nitro und/oder Amino substituiert sein können, abgeleitet ist;

Amino, Monoalkylamino und Dialkylamino mit 1 bis 4 Kohlenstoffatomen je Alkylrest, Monoacylamino, wobei der Acylrest von aliphatischen Carbonsäuren mit 1 bis 7 C-Atomen oder Phenylcarbonsäuren, die im Phenylrest durch OH, Halogen, $C_1$-$C_4$-Alkyl, $C_1$-$C_4$-Alkoxy, Nitro und/oder Amino substituiert sein können, abgeleitet ist;

Mercapto, Alkylthio mit 1 bis 4 Kohlenstoffatomen, Halogen;

Alkylcarbonyl mit 1 bis 4 Kohlenstoffatomen im Alkylrest;

Carboxy, Nitro, Cyan, die Aldehydfunktion, die Sulfonsäuregruppe;

Phthalimido, Pyridyl, Thienyl, Furyl, Isoxyzolyl, Thiazolyl;

einen Phenylrest, der einen oder mehrere gleiche oder verschiedene Substituenten aus der Reihe OH, $NH_2$, $C_1$-$C_4$-Alkyl-NH-, $C_1$-$C_4$-Dialkyl-N-, $C_1$-$C_4$-Alkoxy, $NO_2$, CN, COOH, COO-Alkyl ($C_1$-$C_4$), ($C_1$-$C_6$-Alkyl, Chlor oder Brom, $C_1$-$C_4$-Alkylthio, SH, $C_1$-$C_4$-Alkylsulfonyl, $SO_3H$, $SO_2$-$NH_2$, $SO_2$-NH-Alkyl ($C_1$-$C_4$) tragen kann;

$R^2$, $R^3$ und $R^4$ gleich oder verschieden sind und jeweils für Wasserstoff, $C_1$-$C_6$-Alkyl und Alkenyl, Phenyl, $C_1$-$C_6$-Alkoxy, Phenoxy, $C_1$-$C_6$-Alkylthio, Phenylthio, $C_1$-$C_6$-Alkylsulfonyl, Phenylsulfonyl, Acetyl, Benzoyl, Trifluormethyl, Trifluormethoxy, Trifluormethylthio, Hydroxymethyl, Aminomethyl, $C_1$-$C_6$-Alkylcarbonyloxymethyl, $C_1$-$C_4$-Alkylcarbonylaminomethyl, Benzyl, durch OH, Halogen, $C_1$-$C_4$-Alkyl, $C_1$-$C_4$-Alkoxy, Nitro oder Amino substituiertes Benzyl, Phenethyl, Mercapto, Hydroxy, $C_1$-$C_4$-Alkylcarbonyloxy, Benzoyloxy, Amino, $C_1$-$C_4$-Mono- und Dialkylamino, Acylamino, wobei der Acylrest von aliphatischen Carbonsäuren mit 1 bis 12 C-Atomen oder Phenylcarbonsäuren, die im Phenylrest durch OH, Halogen, $C_1$-$C_4$-Alkyl, $C_1$-$C_4$-Alkoxy, Nitro oder Amino substituiert sein können, abgeleitet ist;

$C_1$-$C_6$-Alkoxycarbonylamino, einen Harnstoff- oder Thioharnstoffrest, wobei das zweite Stickstoffatom durch $C_1$-$C_{12}$-Alkyl, Phenyl oder durch Phenyl, das durch Halogen, $C_1$-$C_4$-Alkyl, $C_1$-$C_4$-Alkoxy, $CF_3$ oder $NO_2$ substituiert ist, substituiert sein kann;

Sulfonylamino, wobei der Sulfonylrest von aliphatischen Sulfonsäuren mit 1 bis 6 C-Atomen oder Phenylsulfonsäuren, deren Phenylrest durch $NO_2$, Amino, Acetamido, $C_1$-$C_4$-Alkyl, $C_1$-$C_4$-Alkoxy, Halogen, $CF_3$ substituiert sein kann, abgeleitet ist;

Carboxyl, Carbalkoxy von $C_1$-$C_{12}$-Alkoholen abgeleitet;

Carbonamido von Ammoniak, von $C_1$-$C_{12}$ aliphatischen, primären oder sekundären Aminen, von Pyrrolidin, Morpholin oder Piperazin, von Anilin, das

durch Halogen, $C_1$-$C_4$-Alkyl, $C_1$-$C_4$-Alkoxy substituiert sein kann;

die Sulfonsäuregruppe, Sulfonamido von $NH_3$, $C_1$-$C_{12}$ primären oder sekundären Aminen, von Pyrrolidin, Piperidin, Piperazin oder Morpholin, von Anilin, das durch Halogen, $C_1$-$C_4$-Alkyl, $C_1$-$C_4$-Alkoxy substi-tuiert sein kann;

F, Cl, Br oder Nitro,

sowie ihre pharmazeutisch verträglichen Salze mit organischen oder anorganischen Säuren, zur Verwendung bei der Bekämpfung von Kreislauferkrankungen.

2. Tricyclische Cytosinderivate der allgemeinen Formel (II)

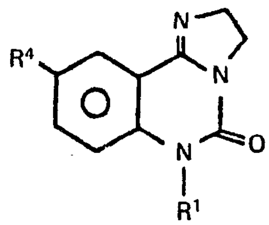

(II)

worin

$R^1$ die in Anspruch 1 angegebene Bedeutung hat und

$R^4$ die folgende Bedeutung hat:

Acylamino, wobei der Acylrest von aliphatischen Carbonsäuren mit 1 bis 12 C-Atomen oder Phenylcarbonsäuren, die im Phenylrest durch OH, Halogen, $C_1$-$C_4$-Alkyl, $C_1$-$C_4$-Alkoxy, Nitro oder Amino substituiert sein können, abgeleitet ist;

$C_1$-$C_6$-Alkoxycarbonylamino, einem Harnstoff- oder Thioharnstoffrest, wobei das zweite Stickstoffatom durch $C_1$-$C_{12}$-Alkyl, Phenyl oder durch Phenyl, das durch Halogen, $C_1$-$C_4$-Alkyl, $C_1$-$C_4$-Alkoxy, $CF_3$ oder $NO_2$ substituiert ist, substituiert sein kann;

Sulfonylamino, wobei der Sulfonylrest von aliphatischen Sulfonsäuren mit 1-6 C-Atomen oder Phenylsulfonsäuren, deren Phenylrest durch $NO_2$, Amino, Acetamido, $C_1$-$C_4$-Alkyl, $C_1$-$C_4$-Alkoxy, Halogen, $CF_3$ substituiert sein kann, abgeleitet ist.

3. Verfahren zur Herstellung von Verbindungen gemäss Anspruch 2, dadurch gekennzeichnet, dass man Anthranilsäuren der allgemeinen Formel (III)

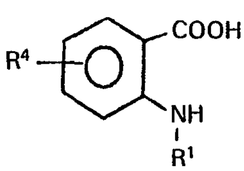

(III)

in welcher

$R^1$ und $R^4$ die in Anspruch 2 angegebene Bedeutung haben,

mit einem Chlorkohlensäureester der allgemeinen Formel (IV)

$$ClCOO\text{-}R^5 \qquad (IV)$$

in welcher

$R^5$ für einen gegebenenfalls substituierten Alkyl-, Aryl- oder Aralkylrest steht,

in Gegenwart von inerten aprotischen Lösungsmitteln und unter Zusatz eines säurebindenden Mittels bei Temperaturen zwischen —20 und 150°C umsetzt und das so erhaltene Reaktionsprodukt nach Entfernung des Lösungsmittels mit einem Überschuss eines Diamins der allgemeinen Formel (V)

$$H_2N\text{-}CH_2\text{-}CH_2\text{-}NH_2 \qquad (V)$$

gegebenenfalls in Gegenwart eines inerten aprotischen Lösungsmittels bei Temperaturen zwischen 20 und 150°C umsetzt und anschliessend nach Entfernen von überschüssigem Diamin der Formel (V) und Lösungsmittel den Rückstand in einem inerten hochsiedenden Lösungsmittel bei Temperaturen zwischen 150 und 250°C cyclisiert, wobei in einem anschliessenden Reaktionsschritt gegebenenfalls solche Verbindungen der allgemeinen Formel (II), in welcher $R^1$ Wasserstoff bedeutet, durch übliche Alkylierungsmethoden mit einem Alkylierungsmittel der allgemeinen Formel (VI)

$$R^1\text{-}Z \qquad (VI)$$

in welcher

$R^1$ die oben angegebene Bedeutung besitzt mit Ausnahme von Wasserstoff und

Z für eine für Alkylierungsmittel übliche austretende Gruppe steht, gegebenenfalls unter Phasentransferbedingungen alkyliert werden.

4. Kreislaufbeeinflussende Arzneimittel, enthaltend mindestens eine Verbindung der allgemeinen Formel (I) Gemäss Anspruch 1.

5. Verfahren zur Herstellung von kreislaufbeeinflussenden Arzneimitteln, dadxurch gekennzeichnet, dass man Verbindungen der allgemeinen Formel (I) gemäss Anspruch 1 gegebenenfalls unter Verwendung von Hilfs- und Trägerstoffen in eine geeignete Applikationsform überführt.

6. Arzneimittel, enthaltend mindestens eine Verbindung der allgemeinen Formel (II) gemäss Anspruch 2.

7. Verfahren zur Herstellung von Arzneimitteln, dadurch gekennzeichnet, dass man Verbindungen der allgemeinen Formel (II) gemäss Anspruch 2 gegebenenfalls unter Verwendung von Hilfs- und Trägerstoffen in eine geeignete Applikationsform überführt.

**Claims**

1. Tricyclic cytosine derivatives of the general formula (I)

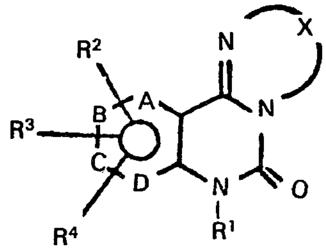

(I)

in which

X represents a saturated bridge member with 2 or

3 carbon atoms which is optionally substituted by 1 or 2 radicals from the series comprising alkyl with 1 to 4 carbon atoms, phenyl or benzyl.

A, B, C and D each represent CH, 1 or 2 of these CH groups optionally being replaced by N,

$R^1$ represents hydrogen, alkyl, alkenyl or alkinyl with in each case up to 18 carbon atoms, it being possible for the alkenyl and alkinyl radicals to contain 1, 2 or 3 double or triple bonds, or a monocyclic, bicyclic or tricyclic aliphatic radical with 3 to 8 carbon atoms, which can be saturated or mono- or di-unsaturated, or a phenyl radical which is optionally substituted by trifluoromethyl, fluorine and nitro or nitro, it being possible for the alkyl and alkenyl radicals mentioned to be substituted by substituents from the following group: hydroxyl, alkoxy with 1 to 4 carbon atoms, phenoxy, it being possible for the phenyl radical to be substituted by $NO_2$, amino, acetamido, $C_1$-$C_4$-alkyl, $C_1$-$C_4$-alkoxy, halogen, $CF_3$, carboxyl or carbalkoxy with 1 to 4 carbon atoms in the alkoxy radical; acyloxy, the acyl radical being derived from aliphatic carboxylic acids with 1 to 7 C atoms or phenylcarboxylic acids which can be substituted in the phenyl radical by OH, halogen, $C_1$-$C_4$-alkyl, $C_1$-$C_4$-alkoxy, nitro and/or amino; amino, monoalkylamino and dialkylamino with 1 to 4 carbon atoms per alkyl radical, monoacylamino, the acyl radical being derived from aliphatic carboxylic acids with 1 to 7 C atoms or phenylcarboxylic acids which can be substituted in the phenyl radical by OH, halogen, $C_1$-$C_4$-alkyl, $C_1$-$C_4$-alkoxy, nitro and/or amino; mercapto, alkylthio with 1 to 4 carbon atoms, and halogen; alkylcarbonyl with 1 to 4 carbon atoms in the alkyl radical; carboxyl, nitro, cyano, the aldehyde funktion, and the sulphonic acid group; phthalimido, pyridyl, thienyl, furyl, isoxyzolyl, and thiazolyl; a phenyl radical which can carry one or more identical or different substituents from the series comprising OH, $NH_2$, $C_1$-$C_4$-alkyl-NH-, $C_1$-$C_4$-dialkyl-N-, $C_1$-$C_4$-alkoxy, $NO_2$, CN, COOH, COO-alkyl ($C_1$-$C_4$), ($C_1$-$C_6$-alkyl, chlorine or bromine, $C_1$-$C_4$-alkylthio, SH, $C_1$-$C_4$-alkylsulphonyl, $SO_3H$, $SO_2$-$NH_2$, and $SO_2$-NH-alkyl ($C_1$-$C_4$);

$R^2$, $R^3$ and $R^4$ are identical or different and each for hydrogen, $C_1$-$C_6$-alkyl and alkenyl, phenyl, $C_1$-$C_6$-alkoxy, phenoxy, $C_1$-$C_6$-alkylthio, phenylthio, $C_1$-$C_6$-alkylsulphonyl, phenylsulphonyl, acetyl, benzoyl, trifluoromethyl, trifluoromethoxy, trifluoromethylthio, hydroxymethyl, aminomethyl, $C_1$-$C_6$-alkylcarbonyloxymethyl, $C_1$-$C_4$-alkylcarbonylaminomethyl, benzyl, benzyl which is substituted by OH, halogen, $C_1$-$C_4$-alkyl, $C_1$-$C_4$-alkoxy, nitro or amino, phenethyl, mercapto, hydroxyl, $C_1$-$C_4$-alkylcarbonyloxy, benzoyloxy,amino, $C_1$-$C_4$-monoalkylamino and dialkylamino, acylamino, the acyl radical being derived from aliphatic carboxylic acids with 1 to 12 C atoms or phenylcarboxylic acids which can be substituted in the phenyl radical by OH, halogen, $C_1$-$C_4$-alkyl, $C_1$-$C_4$-alkoxy, nitro or amino; $C_1$-$C_6$-alkoxycarbonylamino, a urea or thiourea radical, it being possible for the second nitrogen atom to be substituted by $C_1$-$C_{12}$-alkyl, phenyl or by phenyl which is substituted by halogen, $C_1$-$C_4$-alkyl, $C_1$-$C_4$-alkoxy, $CF_3$ or $NO_2$; sulphonylamino, the sulphonyl radical being derived from aliphatic sulphonic acids with 1 to 6 C atoms or phenylsulphonic acids, the phenyl radical of which can be substituted by $NO_2$, amino, acetamido, $C_1$-$C_4$-alkyl, $C_1$-$C_4$-alkoxy, halogen, or $CF_3$; carboxyl and carbalkoxy derived from $C_1$-$C_{12}$-alcohols; carboxamido of ammonia, of $C_1$-$C_{12}$-aliphatic, primary or secondary amines, of pyrrolidine, morpholine or piperazine, and of aniline which can be substituted by halogen, $C_1$-$C_4$-alkyl, or $C_1$-$C_4$-alkoxy; the sulphonic acid group, sulphonamido of $NH_3$, $C_1$-$C_{12}$ primary or secondary amines, of pyrrolidine, piperidine, piperazine or morpholine, and of aniline which can be substituted by halogen, $C_1$-$C_4$-alkyl, or $C_1$-$C_4$-alkoxy; and

F, Cl, Br or nitro,

and their pharmaceutically tolerated salts with organic or inorganic acids, for use in combating circulatory illnesses.

2. Tricyclic cytosine derivatives of the general formula (II)

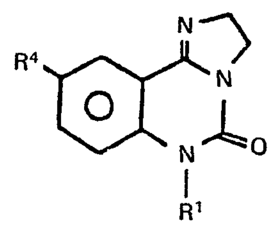
(II)

wherein

$R^1$ has the meaning indicated in claim 1 and

$R^4$ has the following meaning: acylamino, the acyl radical being derived from aliphatic carboxylic acids with 1 to 12 C atoms or phenylcarboxylic acids which can be substituted in the phenyl radical by OH, halogen, $C_1$-$C_4$-alkyl, $C_1$-$C_4$-alkoxy, nitro or amino; $C_1$-$C_6$-alkoxycarbonylamino, or a urea or thiourea radical, it being possible for the second nitrogen atom to be substituted by $C_1$-$C_{12}$-alkyl, phenyl or by phenyl which is substituted by halogen, $C_1$-$C_4$-alkyl, $C_1$-$C_4$-alkoxy, $CF_3$ or $NO_2$; and sulphonylamino, the sulphonyl radical being derived from aliphatic sulphonic acids with 1 to 6 C atoms or phenylsulphonic acids, the phenyl radical of which can be substituted by $NO_2$, amino, acetamido, $C_1$-$C_4$-alkyl, $C_1$-$C_4$-alkoxy, halogen, or $CF_3$.

3. Process for the preparation of compounds according to claim 2, characterised in that anthranilic acids of the general formula (III)

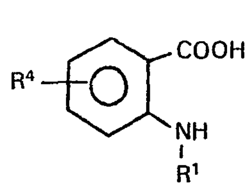
(III)

in which

$R^1$ and $R^4$ have the meaning indicated in claim 2, are reacted with a chlorocarbonic acid ester of the general formula (IV)

$$ClCOO\text{-}R^5 \qquad (IV)$$

in which

$R^5$ represents an optionally substituted alkyl, aryl or aralkyl radical,

in the presence of inert aprotic solvents and with the addition of an acid-binding agent at temperatures between —20 and 150°C and the reaction product thus obtained is reacted, after removal of the solvent, with an excess of a diamine of the general formula (V)

$$H_2N\text{-}CH_2\text{-}CH_2\text{-}NH_2 \quad (V)$$

if appropriate in the presence of an inert aprotic solvent at temperatures between 20 and 150°C and then, after removal of excess diamine of the formula (V) and solvent, the residue is subjected to a cyclisation in an inert high-boiling solvent at temperatures between 150 and 250°C, and, if appropriate, those compounds of the general formula (II) in which $R^1$ denotes hydrogen, are alkylated, in a subsequent reaktion step and by customary alkylating methods, with an alkylating agent of the general formula (VI)

$$R^1\text{-}Z \quad (VI)$$

in which

$R^1$ has the meaning indicated in claim 2, with the exception of hydrogen and

Z represents a group which is a customary departing group for alkylating agents,

if appropriate under phase transfer conditions.

4. Medicaments influencing the circulation, containing at least one compound of the formula (I) according to claim 1.

5. Process for the preparation of medicaments influencing the circulation, characterised in that compounds of the general formule (I) according to claim 1 are converted into a suitable form of administration, if appropriate using auxiliaries and excipients.

6. Medicaments containing at least one compound of the general formula (II) according to claim 2.

7. Process for the preparation of medicaments, characterised in that compounds of the general formula (II) according to claim 2 are converted into a suitable form of administration, if appropriate using auxiliaries and excipients.

**Revendications**

1. Dérivés tricycliques de cytosine de formule générale (I):

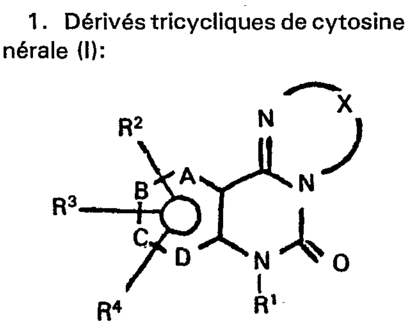

(I)

dans laquelle:

X représente un terme en pont saturé contenant 2 ou 3 atomes de carbone et éventuellement substitué par un ou deux radicaux choisis parmi la série comprenant un groupe alkyle contenant 1 à 4 atomes de carbone, un groupe phényle ou un groupe benzyle,

A, B, C et D représentent chacun CH, un ou deux de ces groupes CH étant éventuellement remplacés par N,

$R^1$ représente un atome d'hydrogène, un groupe alkyle, un groupe alcényle ou un groupe alcinyle contenant chacun jusqu'à 18 atomes de carbone, les groupes alcényle et alcinyle pouvant contenir 1, 2 ou 3 liaisons doubles ou triples, ou un radical aliphatique monocyclique, bicyclique ou tricyclique contenant 3 à 8 atomes de carbone qui peut être saturé ou qui peut comporter une insaturation simple ou double, ou un radical phényle éventuellement substitué par un groupe trifluorométhyle, par un atome de fluor et par un groupe nitro ou par un groupe nitro, les groupes alkyle et alcényle mentionnés pouvant être substitués par des substituants choisis parmi le groupe suivant: un groupe hydroxy, un groupe alkoxy contenant 1 à 4 atomes de carbone, un groupe phénoxy, le radical phényle pouvant être substitué par $NO_2$, un groupe amino, un groupe acétamido, un groupe alkyle en $C_1$-$C_4$, un groupe alcoxy en $C_1$-$C_4$, un atome d'halogène, $CF_3$, un groupe carboxy ou par un groupe carbalcoxy contenant 1 à 4 atomes de carbone dans le radical alcoxy; un groupe acyloxy, le radical acyle dérivant d'acides carboxyliques aliphatiques contenant 1 à 7 atomes de carbone ou d'acides phénylcarboxyliques qui peuvent être substitués dans le radical phényle par OH, un atome d'halogène, un groupe alkyle en $C_1$-$C_4$, un groupe alcoxy en $C_1$-$C_4$, un groupe nitro et/ou par un groupe amino;

un groupe amino, un groupe mono-alkylamino et un groupe dialkylamino contenant 1 à 4 atomes de carbone dans chaque groupe alkyle, un groupe mono-acylamino, le radical acyle dérivant d'acides carboxyliques aliphatiques contenant 1 à 7 atomes de carbone ou d'acides phényl-carboxyliques qui peuvent être substitués dans le radical phényle par OH, un atome d'halogène, un groupe alkyle en $C_1$-$C_4$, un groupe alcoxy en $C_1$-$C_4$, un groupe nitro et/ou par un groupe amino;

un groupe mercapto, un groupe alkylthio contenant 1 à 4 atomes de carbone, un atome d'halogène;

un groupe alkylcarbonyle contenant 1 à 4 atomes de carbone dans le groupe alkyle;

un groupe carboxy, un groupe nitro, un groupe cyano, la fonction aldéhyde, le groupe acide sulfonique;

Un groupe phtalimido, un groupe pyridyle, un groupe thiényle, un groupe furyle, un groupe isoxyzolyle, un groupe thiazolyle;

un radical phényle pouvant comporter un ou plusieurs substituants identiques ou différents choisis parmi la série comprenant un groupe OH, un groupe $NH_2$, un groupe alkyl (en $C_1$-$C_4$)-NH, un groupe dialkyl (en $C_1$-$C_4$)-N-, un groupe alcoxy en $C_1$-$C_4$, un groupe $NO_2$, un groupe CN, un groupe COOH, un groupe COO-alkyle (en $C_1$-$C_4$), un groupe alkyle (en $C_1$-$C_6$), un atome de chlore ou un atome de brome, un groupe alkyl (en $C_1$-$C_4$)-thio, un roupe SH, un groupe alkyl (en $C_1$-$C_4$)-sulfonyle, un groupe $SO_3H$, un groupe $SO_2$-$NH_2$, un groupe $SO_2$-NH-alkyle (en $C_1$-$C_4$);

$R^2$, $R^3$ et $R^4$ sont identiques ou différents et représentent chacun un atome d'hydrogène, un groupe alkyle en $C_1$-$C_6$ et un groupe alcényle, un groupe phényle, un groupe alcoxy en $C_1$-$C_6$, un groupe phénoxy, un groupe alkyl (en $C_1$-$C_6$)-thio, un groupe phénylthio, un groupe alkyl (en $C_1$-$C_6$)-sulfonyle, un groupe phénylsulfonyle, un groupe acétyle, un groupe benzoyle, un groupe trifluorométhyle, un groupe trifluorométhoxy, un groupe trifluorométhylthio, un groupe hydroxyméthyle, un groupe aminométhyle, un groupe alkyl (en $C_1$-$C_6$)-carbonyloxyméthyle, un groupe alkyl (en $C_1$-$C_4$)-carbonylaminométhyle, un groupe benzyle, un groupe benzyle substitué par un groupe OH, un atome d'halogène, un groupe alkyle en $C_1$-$C_4$, un groupe alcoxy en $C_1$-$C_4$, un groupe nitro ou par un groupe amino, un groupe phénéthyle, un groupe mercapto, un groupe hydroxy, un groupe alkyl (en $C_1$-$C_4$)-carbonyloxy, un groupe benzoyloxy, un groupe amino, un groupe mono- et dialkyl (en $C_1$-$C_4$)amino, un groupe acylamino, le radical acyle dérivant d'acides carboxyliques aliphatiques contenant 1 à 12 atomes de carbone ou d'acides phénylcarboxyliques qui peuvent être substitués dans le radical phényle par un groupe OH, un atome d'halogène, un groupe alkyle en $C_1$-$C_4$, un groupe alcoxy en $C_1$-$C_4$, un groupe nitro ou par un groupe amino;

un groupe alcoxy (en $C_1$-$C_6$)-carbonylamino, un radical urée ou thiourée, le deuxième atome d'azote pouvant être substitué par un groupe alkyle en $C_1$-$C_{12}$, par un groupe phényle ou par un groupe phényle qui est substitué par un atome d'halogène, un groupe alkyle en $C_1$-$C_4$, un groupe alcoxy en $C_1$-$C_4$, un groupe $CF_3$ ou par $NO_2$;

un groupe sulfonylamino, le radical sulfonyle dérivant d'acides sulfoniques aliphatiques contenant 1 à 6 atomes de carbone ou d'acides phénylsulfoniques dont le radical phényle peut être substitué par $NO_2$, un groupe amino, un groupe acétamido, un groupe alkyle en $C_1$-$C_4$, un groupe alcoxy en $C_1$-$C_4$, un atome d'halogène, ou par $CF_3$;

un groupe carboxy, un groupe carbalcoxy dérivant d'alcools en $C_1$-$C_{12}$;

un groupe carbonamido d'ammoniac, d'amines aliphatiques primaires ou secondaires en $C_1$-$C_{12}$, de pyrrolidine, de morpholine ou de pipérazine, ainsi que d'aniline pouvant être substituée par un atome d'halogène, par un groupe alkyle en $C_1$-$C_4$ ou par un groupe alcoxy en $C_1$-$C_4$;

le groupe acide sulfonique, un groupe sulfonamido de $NH_3$, d'amines primaires ou secondaires en $C_1$-$C_{12}$, de pyrrolidine, de pipéridine, de pipérazine ou de morpholine, ainsi que d'aniline pouvant être substituée par un atome d'halogène, par un groupe alkyle en $C_1$-$C_4$ ou par un groupe alcoxy en $C_1$-$C_4$; F, Cl, Br ou le groupe nitro,

ainsi que leurs sels pharmaceutiquement compatibles avec des acides organiques ou inorganiques, en vue de les utiliser pour combattre les maladies de la circulation.

2. Dérivés tricycliques de cytosine de formule générale (II):

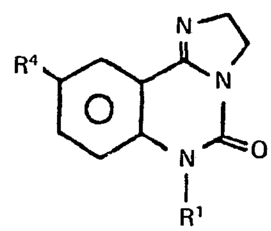

(II)

dans laquelle

$R^1$ a la signification indiquée dans la revendication 1, et

$R^4$ a la signification suivante:

un groupe acylamino, le radical acyle dérivant d'acides carboxyliques aliphatiques contenant 1 à 12 atomes de carbone ou d'acides phénylcarboxyliques qui peuvent être substitués dans le radical phényle par un groupe OH, un atome d'halogène, un groupe alkyle en $C_1$-$C_4$, un groupe alcoxy en $C_1$-$C_4$, un groupe nitro ou par un groupe amino;

un groupe alcoxy (en $C_1$-$C_6$) carbonylamino, un radical urée ou thiourée, le deuxième atome d'azote pouvant être substitué par un groupe alkyle en $C_1$-$C_{12}$. par un groupe phényle ou par un groupe phényle qui est substitué par un atome d'halogène, un groupe alkyle en $C_1$-$C_4$, un groupe alcoxy en $C_1$-$C_4$, $CF_3$ ou par $NO_2$;

un groupe sulfonylamino, le radical sulfonyle dérivant d'acides sulfoniques aliphatiques contenant 1 à 6 atomes de carbone ou d'acides phénylsulfoniques dont le radical phényle peut être substitué par un groupe $NO_2$, un groupe amino, un groupe acétamido, un groupe alkyle en $C_1$-$C_4$, un groupe alcoxy en $C_1$-$C_4$, un atome d'halogène ou par $CF_3$.

3. Procédé de préparation de composés suivant la revendication 2, caractérisé en ce qu'on fait réagir des acides anthraniliques de formule générale (III):

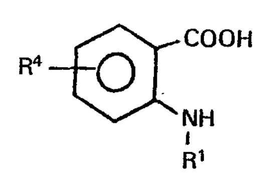

(III)

dans laquelle

$R^1$ et $R^4$ ont la signification indiquée dans la revendication 2,

avec un ester d'acide chlorocarbonique de formule générale (IV):

$$ClCOO\text{-}R^5 \qquad (IV)$$

dans laquelle

$R^5$ représente un groupe aralkyle, un groupe aryle ou un groupe alkyle éventuellement substitué,

en présence de solvants aprotiques inertes et avec addition d'un agent fixateur d'acide à des températures comprises entre —20 et 150°C et, après

élimination du solvant, on fait réagir le produit réactionnel ainsi obtenu avec un excés d'une diamine de formule générale (V):

$$H_2N\text{-}CH_2\text{-}CH_2\text{-}NH_2 \qquad (V)$$

éventuellement en présence d'un solvant aprotique inerte à des températures comprises entre 20 et 150°C puis, après élimination de la diamine en excés de formule (V) et du solvant, on cyclise le résidu dans un solvant inerte à point d'ébullition élevé à des températures comprises entre 150 et 250°C, ces composés de formule générale (II) dans laquelle $R^1$ représente un atome d'hydrogène, étant éventuellement alkylés dans une étape réactionnelle ultérieure et par des méthodes habituelles d'alkylation avec un agent d'alkylation de formule générale (VI):

$$R^1\text{-}Z \qquad (VI)$$

dans laquelle

$R^1$ a la signification indiquée dans la revendication 2, à l'exception de l'hydrogène, et

Z représente un groupe habituel qui se sépare pour l'agent d'alkylation, éventuellement dans des conditions de transfert de phases.

4. Médicaments influençant la circulation, ces médicaments contenant au moins un composé de formule générale (I) suivant la revendication 1.

5. Procédé de préparation de médicaments influençant la circulation, caractérisé en ce qu'on transforme des composés de formule générale (I) suivant la revendication 1 en une forme d'application appropriée en utilisant éventuellement des substances auxiliaires et des substances supports.

6. Médicaments contenant au moins un composé de formule générale (II) suivant la revendication 2.

7. Procédé de préparation de médicaments, caractérisé en ce qu'on transforme des composés de formule générale (II) suivant la revendication 2 en une forme d'application appropriée en utilisant éventuellement des substances auxiliaires et des substances supports.